# EUROPEAN PATENT APPLICATION

(11) **EP 3 912 988 A1**
(43) Date of publication of application: **24.11.2021**
(21) Application number: 20741653.8
(22) Date of filing: 14.01.2020
(51) Int. Cl.: C07K 14/005, C07K 16/10, A61K 39/29, A61P 31/14

(54) **THREE-DIMENSIONAL EPITOPE OF HEPATITIS B SURFACE ANTIGEN AND ANTIBODY BINDING SPECIFICALLY THERETO**

(30) Priority: 17.01.2019 KR 20190006092
(71) Applicant: Green Cross Corporation, Yongin-si, Gyeonggi-do 16924 (KR)
(72) Inventor: KIM, Jung-Hwan, Gyeonggi-do 16924 (KR); KIM, Woohyun, Gyeonggi-do 16924 (KR)
(74) Representative: HGF
(86) International application number: PCT/KR2020/000683
(87) International publication number: WO 2020/149615

(57) **Abstract**

The present invention relates to a specific three-dimensional epitope of a hepatitis B surface antigen and a hepatitis B neutralizing antibody binding thereto. The epitope provided by the present invention has a specific three-dimensional structure. In addition, the three-dimensional epitope of the present application does not contain the 'a' determinant that may generate an escape mutation upon administration of conventional vaccines or HBIg. Thus, an antibody capable of binding to the three-dimensional epitope of the present application is highly unlikely to allow the emergence of a vaccine escape mutation, which is caused by conventional vaccines, and as such, can retain a sustained effect. Therefore, such an antibody or a vaccine composition can find effective applications in the prevention and treatment of HBV, having great economic value.

## Description

### Technical Field

The present invention relates to a conformational epitope of hepatitis B surface antigen (hereinafter referred to as HBsAg) and an antibody binding specifically thereto.

### Background Art

Patients with chronic hepatitis B have no or very poor T cell responses as compared with spontaneously healed patients. It has been reported that this is because T cells specific to HBV have disappeared or failed to function properly as a result of continuous exposure to excessive amounts of viral antigens such as hepatitis B surface antigen (HBsAg).

For chronic hepatitis B patients, viral particles in the blood reach 10¹⁰ particles/ml, and HBsAgs may reach even a level of 100 ug/ml. Such an excessive amount of virus particles or HBsAgs suppresses the human body's immune function, and thus is thought to be an important cause of chronic hepatitis. In addition, it has been reported that HBV particles or HBsAgs can enter dendritic cells to decrease activation of T cells, B cells, and NK cells. In addition, in an experiment using dendritic cells isolated from the healthy human's blood, it was observed that a recombinant hepatitis B surface antigen (rHBsAg) entered dendritic cells, and these dendritic cells had decreased expression of activation marker proteins. In addition, when these dendritic cells were co-cultured with NK cells and T cells, such dendritic cells also had a decreased ability to activate cells such as NK cells and T cells (Woltman AM, PLos ONE, e15324, January 5, 2011).

This phenomenon, in which loss of immune function or induction of immune tolerance occurs, was also observed in mice infected with adeno-associated virushepatitis B virus (AAV-HBV). Interestingly, in a case where an anti-HBsAg monoclonal antibody is injected to decrease an HBsAg concentration in the blood, it has been shown that the phenomenon of immune tolerance gradually decreased to restore the function of B cells and helper T cells.

Meanwhile, it is known that most of the antibodies produced by conventional hepatitis B vaccination recognize the 'a' determinant, which is a site between amino acids 124 to 147 of HBsAg. In addition, it has been reported that although the 'a' determinant acts as a major neutralizing epitope of HBV, certain mutants having a mutation within the 'a' determinant, which have appeared in some patients, can evade the antibodies produced by hepatitis B vaccination.

Therefore, there is a growing need for development of novel antibodies or vaccines for preventing and treating hepatitis B, which can counteract such escape mutants generated against existing vaccines or hepatitis B immunoglobulins (HBIgs). In particular, to produce and verify an effective antibody against HBsAg, there is a need to identify the exact epitope of the antigen.

### Disclosure of Invention

### Technical Problem

An object of the present invention is to provide a conformational epitope of hepatitis B surface antigen, and an antibody or a fragment thereof which specifically binds to the epitope and has excellent binding capacity to various mutant HBsAgs.

### Solution to Problem

To achieve the above-mentioned object, in an aspect of the present invention, there is provided a conformational epitope of hepatitis B surface antigen (HBsAg), and a hepatitis B virus-neutralizing antibody or a fragment thereof which specifically binds to the epitope.

### Advantageous Effects of Invention

The conformational epitope of hepatitis B surface antigen (HBsAg) provided herein contains all of the key residues, which are important for specific binding to a hepatitis B virus-neutralizing antibody, and maintains an appropriate three-dimensional structure, which allows the epitope to show high affinity to the hepatitis B virus-neutralizing antibody. Accordingly, based on its high immunogenicity, the epitope of the present invention can be used as an excellent HBV vaccine composition. In addition, the HBV neutralizing antibody produced by using the epitope of the present invention can effectively eliminate HBsAg present in the blood, and induce recovery of immunity in an individual infected with hepatitis B virus, thereby effectively treating hepatitis B. In addition, the antibody can effectively bind to various HBsAg variants, thereby effectively eliminating hepatitis B virus.

### Brief Description of Drawings

FIG. 1 illustrates a schematic diagram of the structure of HBsAg.
FIG. 2 illustrates results obtained by analyzing characteristics of HBsAg viruslike particle (VLP) on a native agarose gel.
FIG. 3 illustrates an analysis method using denatured HBsAg VLP and an HBsAg VLP-specific antibody (GC-100A).
FIG. 4 illustrates that the denatured HBsAg VLP does not bind to the HBsAg VLP-specific antibody (GC-100A). Here, N means a native condition, and D means a denatured condition.
FIG. 5 illustrates a schematic diagram of the binding site on the conformational epitope of HBsAg VLP which binds to the HBsAg VLP-specific antibody (GC-100A).
FIGS. 6 to 9 illustrate, through native immunoblotting, the degree of binding between the HBsAg VLP-specific antibody (GC-100A) and 30 point-mutated HBsAgs, relating to 16 major amino acid residues, which are known as vaccine escape mutants.
FIG. 10 illustrates binding profiles of GC-100A and DAKO antibodies to clinical ('a' determinant) variant HBsAgs.

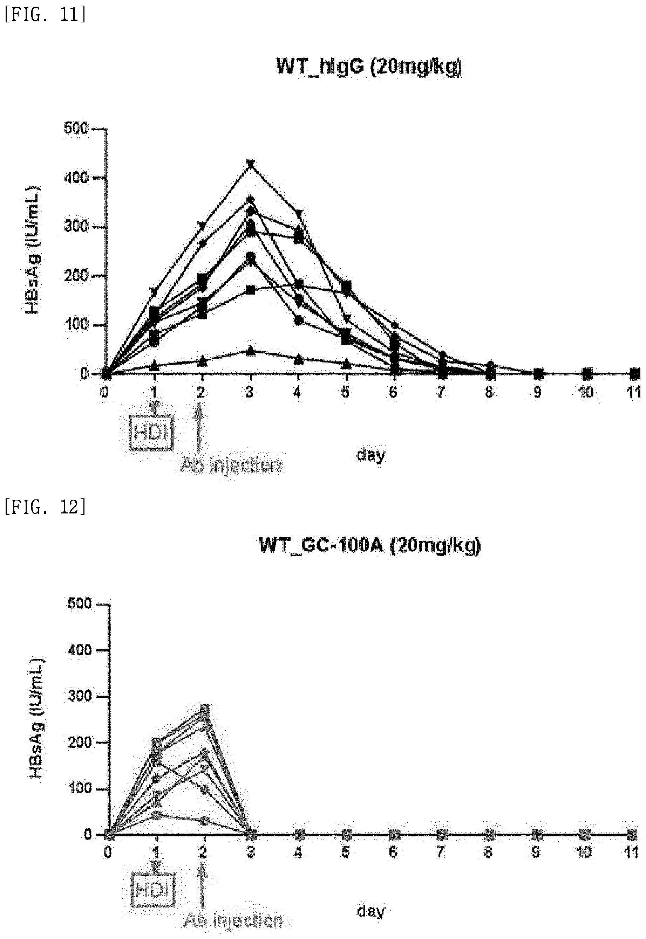

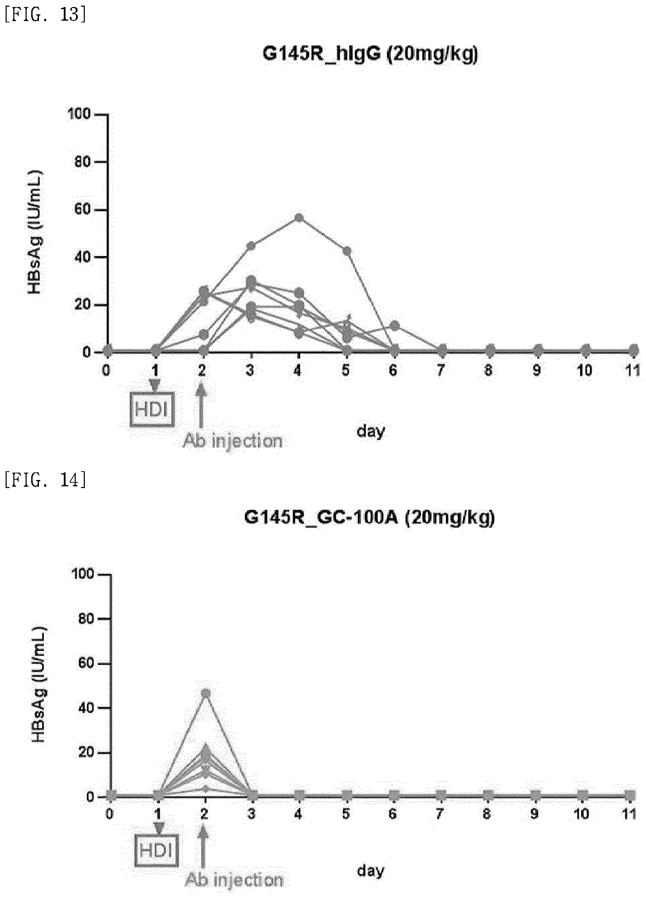

FIGS. 11 to 14 illustrate results obtained by performing ELISA on HBsAg in a wild-type virus and a vaccine escape mutant (G145R) in an HBV short-term expression mouse model. Specifically, FIG. 11 shows a binding reaction with hIgG in mice infected with HBV. In addition, FIG. 12 shows results which identify that the HBsAg VLP-specific antibody (GC-100A) binds well to HBsAg VLP produced in mice infected with HBV. In addition, FIG. 13 shows a binding reaction with hIgG in wild-type mice infected with a vaccine escape mutant (G145R) of HBV In addition, FIG. 14 shows results which identify that the HBsAg VLP-specific antibody (GC-100A) binds well to HBsAg VLP produced in wild-type mice infected with a vaccine escape mutant (G145R) of HBV Here, the Y-axis means a concentration of HBsAg in the blood, which is expressed in IU per mL. The X-axis means days. On day 1, hydrodynamic injection (injection of DNA) was performed; and on day 2, administration of IgG, which is a control, or GC-100A was performed. Each line in the same experiment represents each individual.

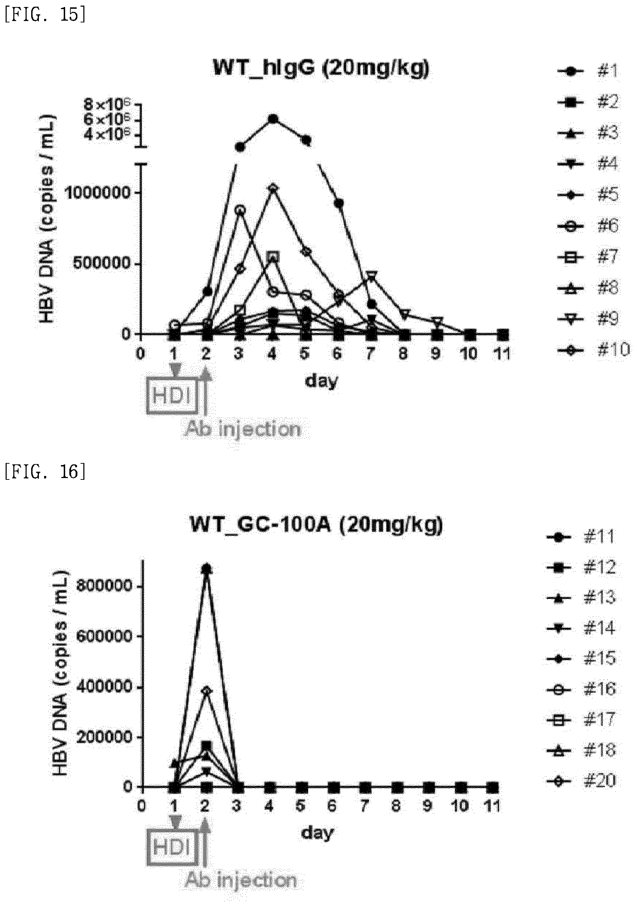

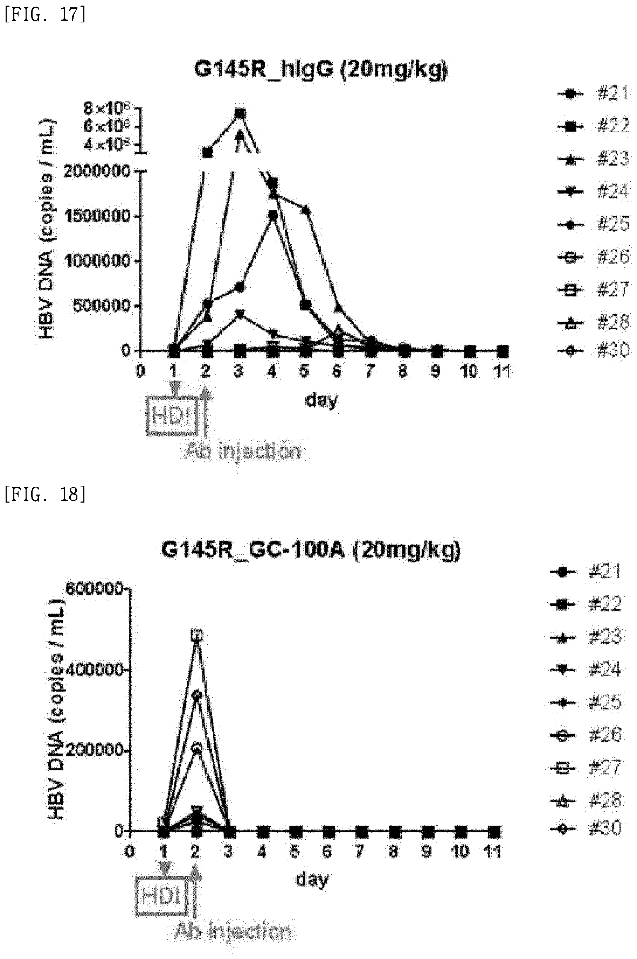

FIGS. 15 to 18 illustrate results which identify, through quantification of HBV DNA, capacity of eliminating a wild-type virus and a vaccine escape mutant (G145R) in an HBV short-term expression mouse model. Specifically, FIG. 15 shows results which identify that HBV is not eliminated by hIgG in mice infected with HBV. In addition, FIG. 16 shows results which identify that HBV is effectively eliminated by GC-100A in mice infected with HBV. In addition, FIG. 17 shows results which identify that HBV is not eliminated by hIgG in wild-type mice infected with a vaccine escape mutant (G145R) of HBV In addition, FIG. 18 shows results which identify that HBV is effectively eliminated by GC-100A in wild-type mice infected with a vaccine escape mutant (G145R) of HBV Here, the Y-axis means a concentration of HBV DNA in the blood, which is expressed in number of copies per mL. The X-axis means days. On day 1, hydrodynamic injection (injection of DNA) was performed; and on day 2, administration of IgG, which is a control, or GC-100A was performed. Each line in the same experiment represents each individual.
FIGS. 19 to 21 illustrate results obtained by performing native immunoblotting on HBsAg for each genotype/serotype.
FIG. 22 illustrates binding profiles of GC-100A and DAKO antibodies to HBsAg for each genotype/serotype.
FIG. 23 illustrates a diagram representing the geographic distribution of each HBV genotype.
FIGS. 24 to 26 illustrate results obtained by performing native immunoblotting on HBsAg mutants for each genotype/serotype.
FIG. 27 illustrates binding profiles of GC-100A and DAKO antibodies to HBsAg for each genotype/serotype.

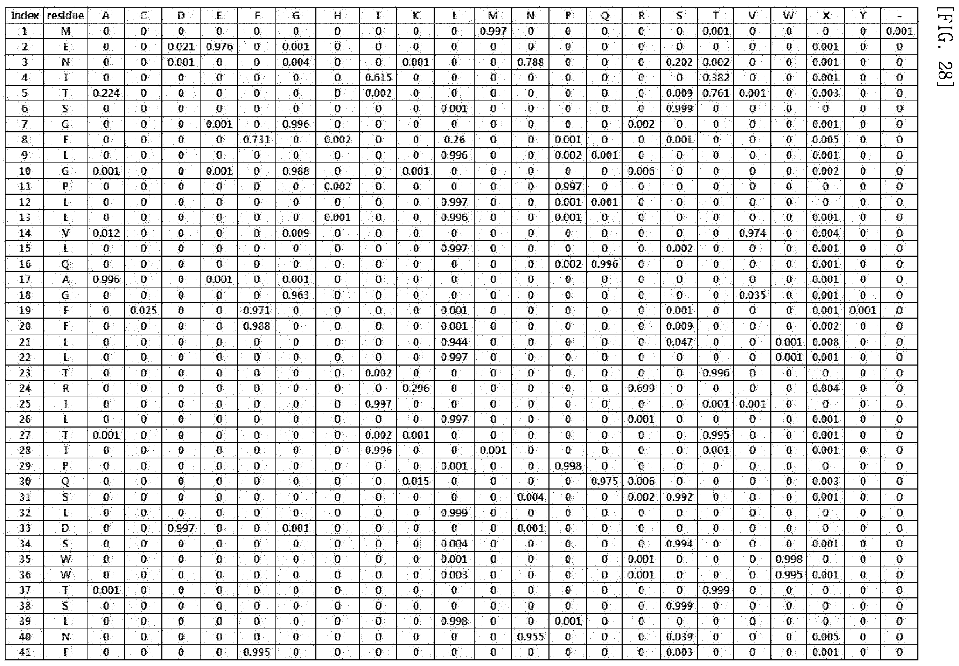

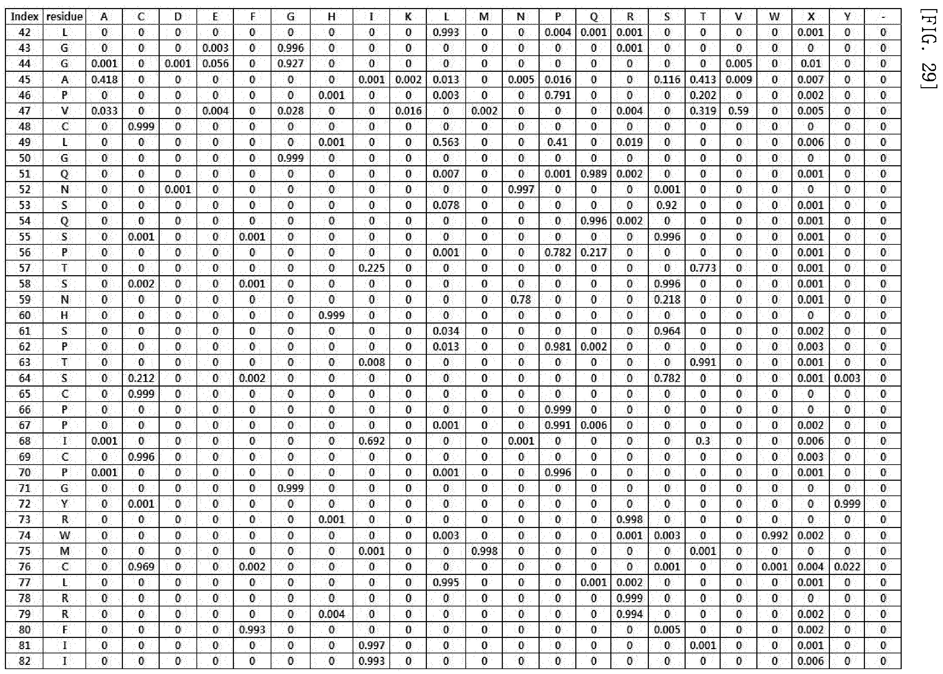

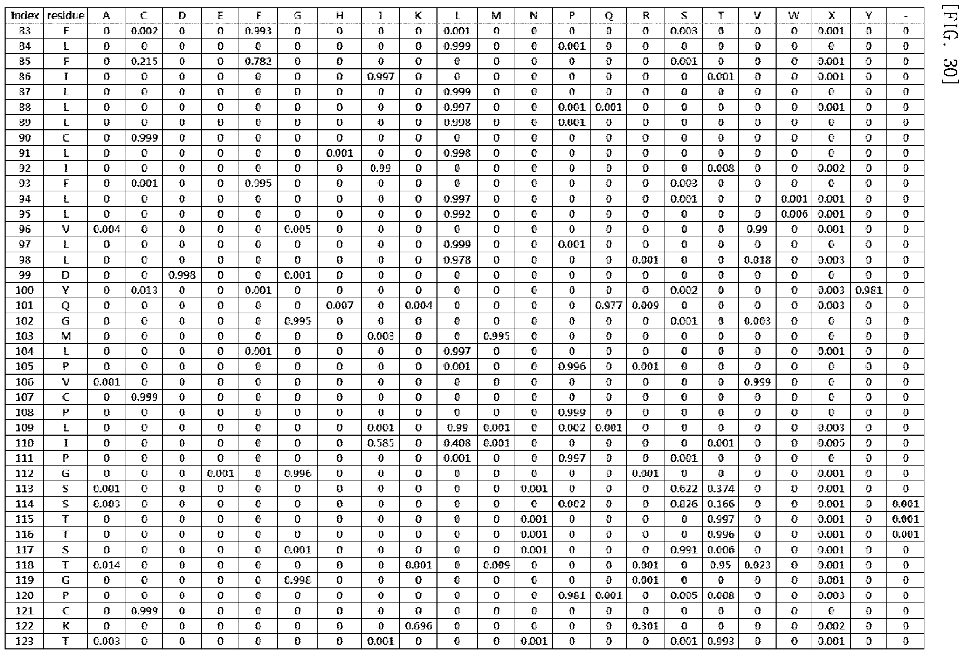

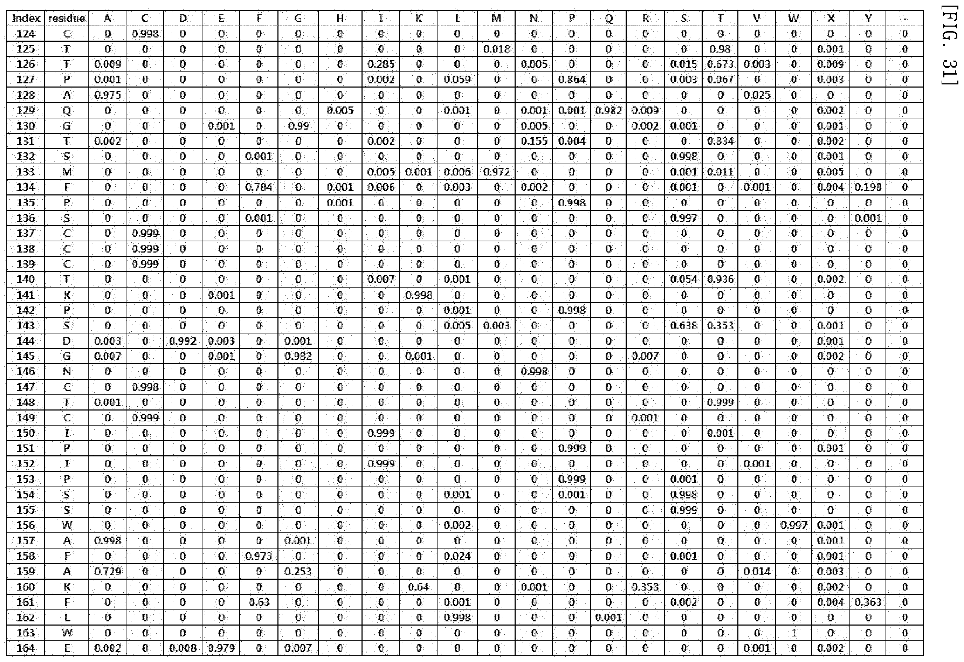

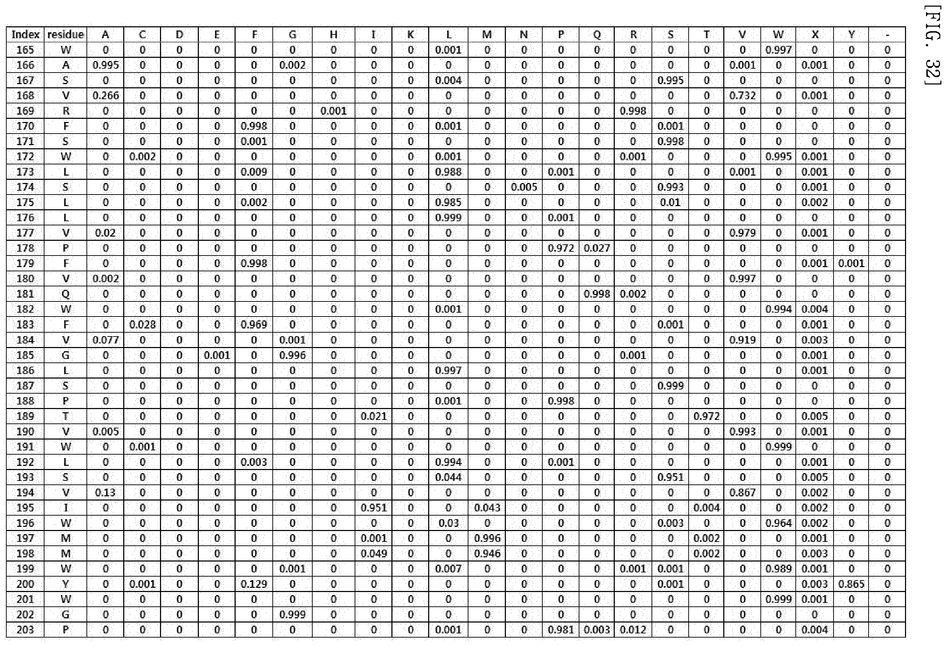

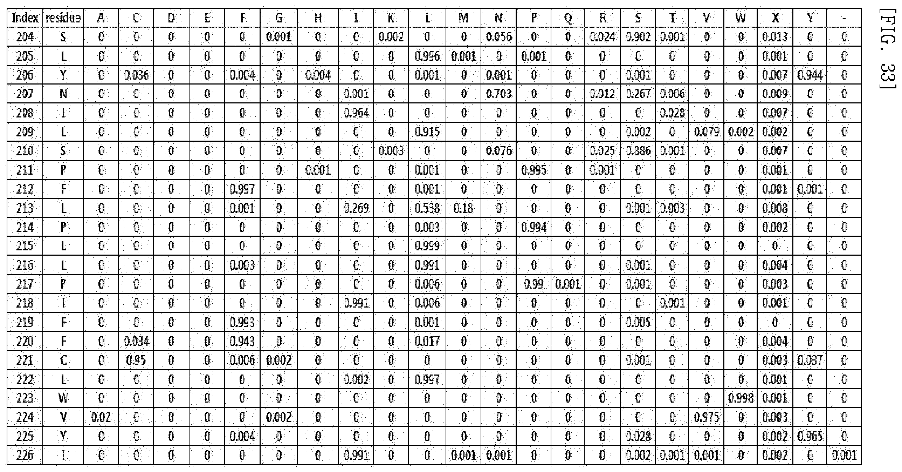

FIGS. 28 to 33 illustrate results of homology comparison analysis on various amino acid sequences of hepatitis B surface antigen. The horizontal axis represents distribution of amino acids that may exist in the surface antigen. The vertical axis represents representative amino acids depending on positions of the surface antigen.

### Best Mode for Carrying out the Invention

Hereinafter, the present invention will be described in detail.

In an aspect of the present invention, there is provided a conformational epitope of hepatitis B surface antigen (HBsAg), comprising a peptide represented by General Formula 1,

**General Formula 1** Thr-(X₁)ₙ₁-A₁-A₂-(X₂)ₙ₂-A₃-(X₃)ₙ₃-A₄-(X₄)ₙ₄-A₅-(X₅)ₙ₅-A₆

in the general formula, A₁ is Lys or Arg; A₂ is Thr, Ala, Ile, Asn, or Ser; A₃ is Ser or Leu; A₄ is Arg or His; A₅ is Ser or Phe; and A₆ is Ser or Asn; and
X₁ to X₅ are each independently a peptide molecule formed by bonding of n1 to n5 identical or different amino acids to each other, in which n1 is an integer from 4 to 8; n2 is an integer from 41 to 45; n3 is an integer from 0 to 2; n4 is an integer from 0 to 2; and n5 is an integer from 0 to 4.

According to an embodiment, n1 may be an integer of 4, 5, 6, 7, or 8, and may be preferably an integer of 6. n2 may be an integer of 41, 42, 43, 44, or 45, and may be preferably an integer of 43. n3 may be an integer of 0, 1, or 2, and may be preferably 1. n4 may be an integer of 0, 1, or 2, and may be preferably 1. n5 may be 0, 1, 2, 3, or 4, and may be preferably 2.

Here, X₁ to X₅ may be each independently composed of amino acids selected from the group consisting of Ala, Arg, Asn, Asp, Cys, Glu, Gly, His, Ile, Leu, Lys, Met, Phe, Pro, Ser, Thr, Trp, Tyr, and Val.

The present inventors have conducted studies to identify a specific epitope, which shows excellent immunogenicity, in HBsAg. As a result, the present inventors have found that the seven amino acid residues (Thr and A₁ to A₆), which are represented by General Formula 1, in HBsAg play an important role in specific binding to a hepatitis B virus-neutralizing antibody, and that a peptide molecule, which contains the 7 amino acid residues and maintains an appropriate three-dimensional structure, can be used as an excellent HBV vaccine composition, thereby completing the present invention.

As used herein, the term "hepatitis B virus" refers to a DNA virus that belongs to the family Hepadnaviridae and has a double helix structure of nucleotides which is about 3.2 kb in size. Hepatitis B virus has four genes, that is, pre-core/core, pre-S/S, P, and X. These genes encode HBeAg/HBcAg, HBsAg, DNA polymerase, and HBx protein. The nucleotide sequence constituting HBV has wide variations depending on regions and races. Depending on such nucleotide sequence variations, HBV serotypes are expressed differently. For such serotypes, the serotype adw is subdivided into subtypes adw, adw2, and adw4; and the serotype adr or ayw is also further subdivided in a similar way. HBV in Korea was found to be serotype adr.

As used herein, the term "hepatitis B surface antigen" refers to a protein present on an outer coat of hepatitis B virus, and is also designated as HBsAg. Here, the hepatitis B surface antigen (HBsAg) may be a protein composed of the amino acids of SEQ ID NO: 1. The antigen may have a linear epitope having a one-dimensional structure and a conformational epitope having a three-dimensional structure. Usually, the linear epitope is composed of contiguous amino acids.

According to an embodiment of the present invention, in General Formula 1, A₁ is Lys or Arg; A₂ is Thr; A₃ is Ser; A₄ is Arg; A₅ is Ser; and A₆ is Ser or Asn. More specifically, A₁ is Lys, and A₆ is Ser.

In such cases, the conformational epitope of HBsAg may have any one of the structures of General Formulas 2 to 5:

**General Formula 2** Thr-(X₁)ₙ₁-Lys-Thr-(X₂)ₙ₂-Ser-(X₃)ₙ₃-Arg-(X₄)ₙ₄-Ser-(X₅)ₙ₅-Ser

**General Formula 3** Thr-(X₁)ₙ₁-Lys-Thr-(X₂)ₙ₂-Ser-(X₃)ₙ₃-Arg-(X₄)ₙ₄-Ser-(X₅)ₙ₅-Asn

**General Formula 4** Thr-(X₁)ₙ₁-Arg-Thr-(X₂)ₙ₂-Ser-(X₃)ₙ₃-Arg-(X₄)ₙ₄-Ser-(X₅)ₙ₅-Ser

**General Formula 5** Thr-(X₁)ₙ₁-Arg-Thr-(X₂)ₙ₂-Ser-(X₃)ₙ₃-Arg-(X₄)ₙ₄-Ser-(X₅)ₙ₅-Asn

According to a specific embodiment of the present invention, n1 is 6; n2 is 43; n3 is 1; n4 is 1; and n5 is 2. Here, specifically, (X₁)₆ may have the amino acid sequence represented by SEQ ID NO: 2. In addition, (X₂)₄₃ may have the amino acid sequence represented by SEQ ID NO: 3. In addition, (X₃)₁ may be Ser. In addition, (X₄)₁ may be Arg. In addition, (X₅)₂ may be Trp-Leu.

According to an embodiment of the present invention, the HBsAg conformational epitope of the present invention may be at any one selected from the group consisting of HBsAg amino acid positions 115, 122, 123, 167, 169, 171, and 174. Specifically, the epitope may include all seven amino acids. Specifically, the conformational epitope of hepatitis B surface antigen (HBsAg) may be an HBsAg conformational epitope composed of a 7-mer- to 60-mer oligomer whose residues are at amino acid positions 115 to 174 of hepatitis B surface antigen (HBsAg), and the HBsAg conformational epitope may include amino acid residues at positions 115, 122, 123, 167, 169, 171, and 174. Here, the hepatitis B surface antigen (HBsAg) may have the amino acid sequence represented by SEQ ID NO: 1.

In another aspect of the present invention, there is provided an HBV vaccine, comprising the HBsAg conformational epitope as an active ingredient. Here, the HBsAg conformational epitope, which is included as the active ingredient in the HBV vaccine, is characterized by being native. The epitope containing the amino acids present at the above-mentioned positions may be used in combination with a carrier in order to maintain its three-dimensional structure or cause increased efficiency in a vaccine composition. Here, for the carrier according to the present invention, any carrier may be used as long as it is biocompatible and can achieve a desired effect in the present invention. The carrier may be selected from, but is not limited to, serum albumin, peptide, immunoglobulin, hemocyanin, polysaccharide, and the like.

In yet another aspect of the present invention, there is provided a hepatitis B virus-neutralizing antibody or a fragment thereof, which specifically binds to the conformational epitope of hepatitis B surface antigen (HBsAg) of the present invention as described above. Specifically, the hepatitis B virus-neutralizing antibody or the fragment thereof may specifically bind to the above-described HBsAg conformational epitope at HBsAg amino acid positions 115, 122, 123, 167, 169, 171, and 174.

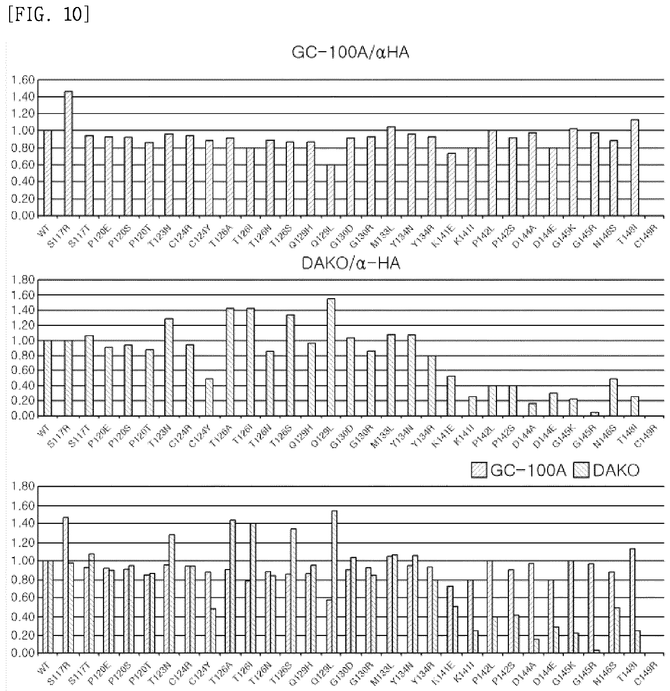

Here, the neutralizing antibody or the fragment thereof may have a therapeutic effect on infection with a vaccine escape mutant (see FIG. 10).

Here, the antibody may be a monoclonal antibody. In addition, the antibody may be produced in a cell line having accession number KCTC13760BP. In addition, in a case where an antibody has the same epitope (complementarity-determining region, CDR) as the antibody produced in the above-mentioned deposited strain, it can be determined that such an antibody belongs to the antibody disclosed in the present invention. In addition, the antibody's fragment may be any one selected from the group consisting of Fab, sFv, and F(ab')₂, and may be a diabody or a chimeric antibody. However, the fragment is not limited thereto.

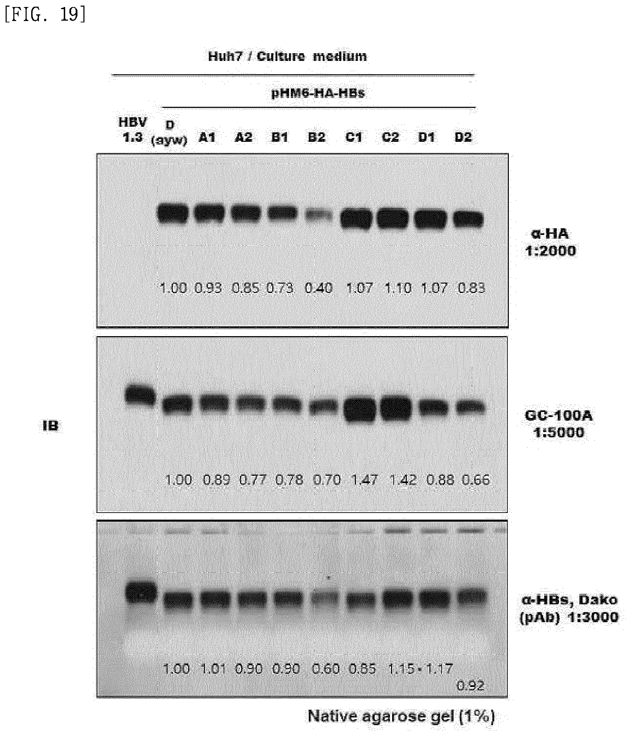

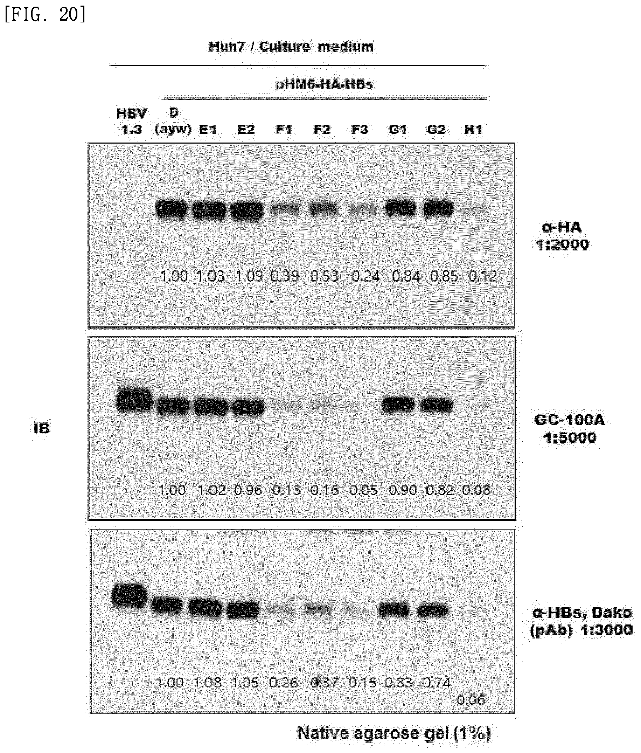

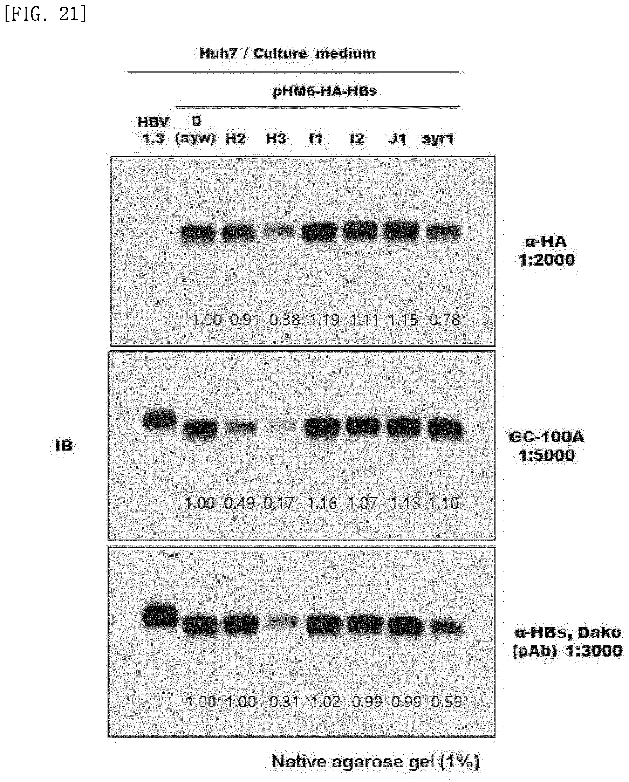

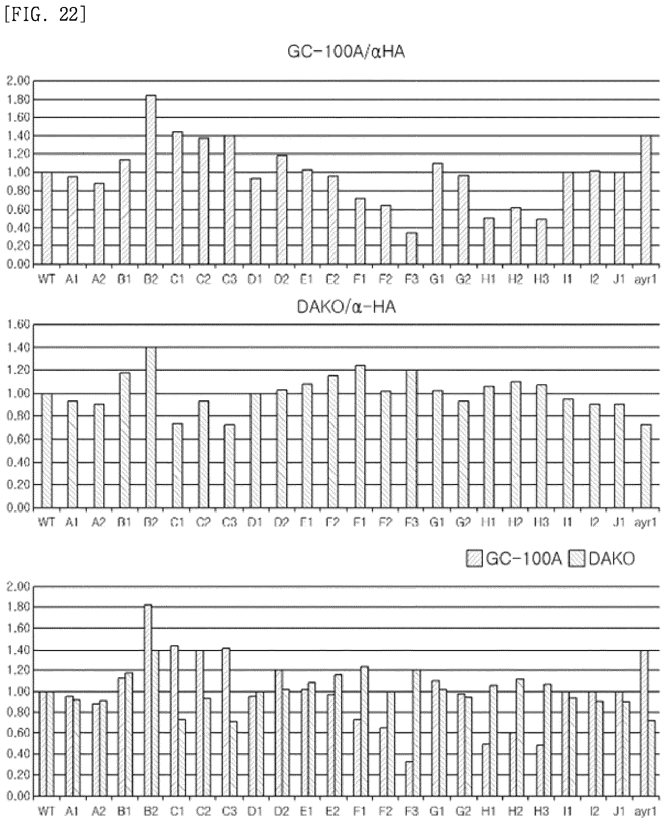

The antibody or the fragment thereof, according to the present invention, may bind to hepatitis B virus whose genotype is A, B, C, D, E, F, G, or H, and thus have neutralizing activity thereagainst. In addition, the antibody or the fragment thereof may bind to any one or more selected from the group consisting of the hepatitis B surface antigen (HBsAg) subtypes adw, adr, ayw, and ayr, and thus have neutralizing activity against hepatitis B virus (Experimental Example 3.3 and FIGS. 19 to 22).

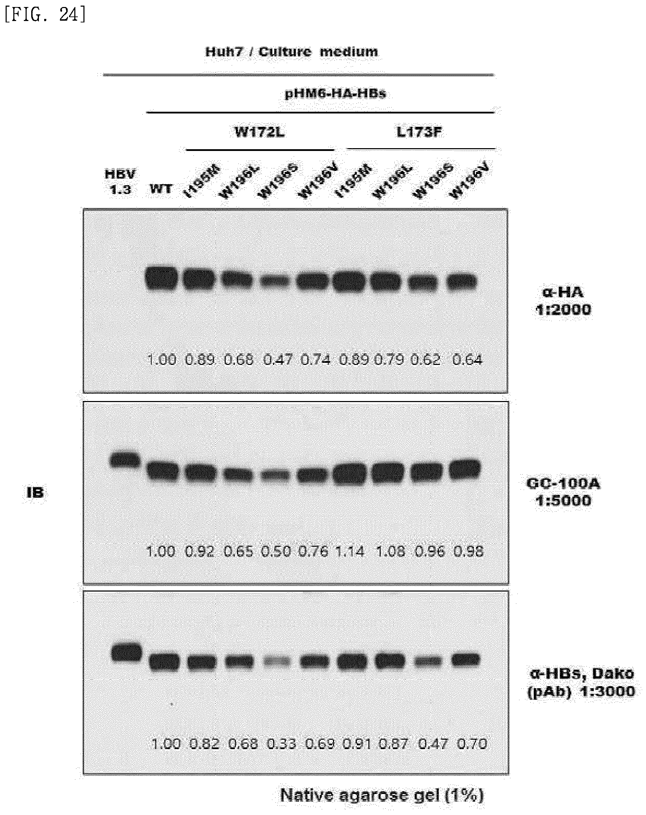

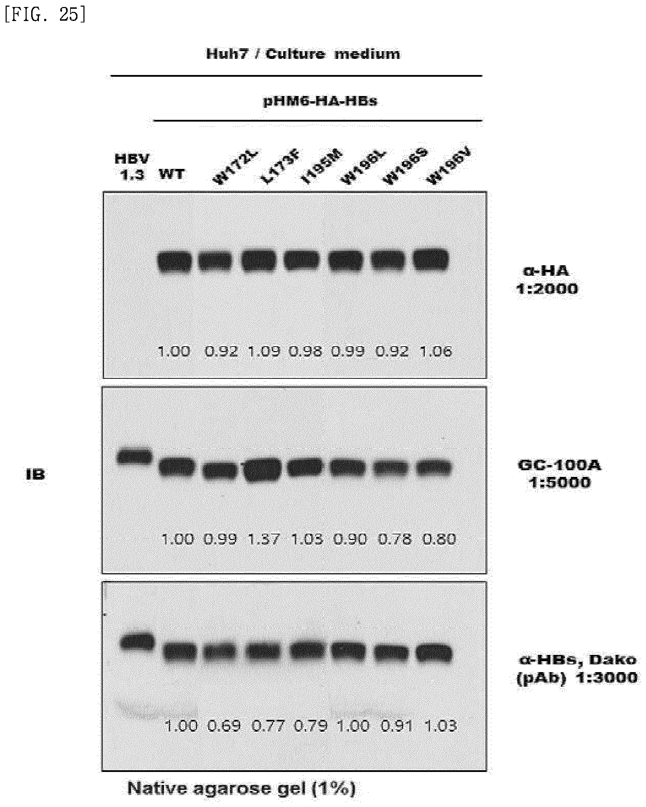

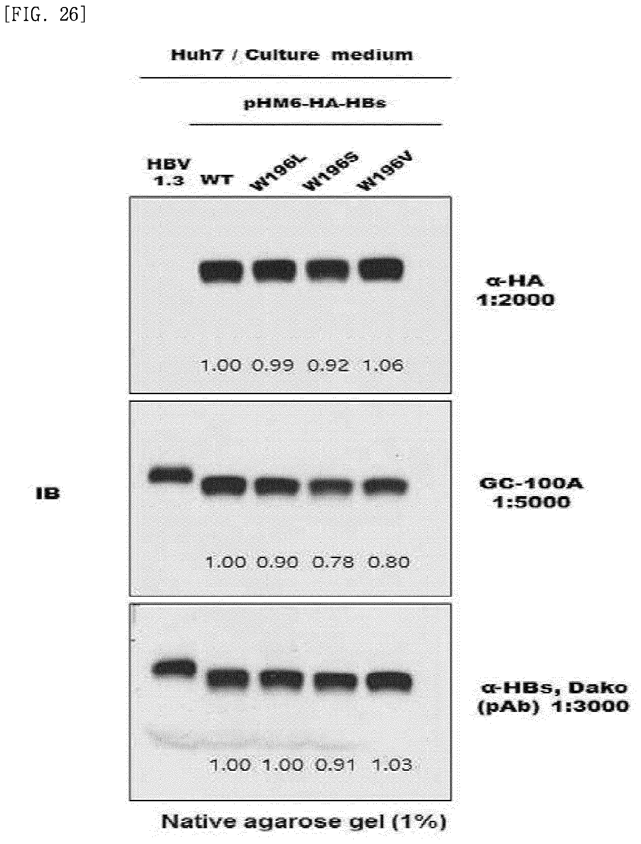

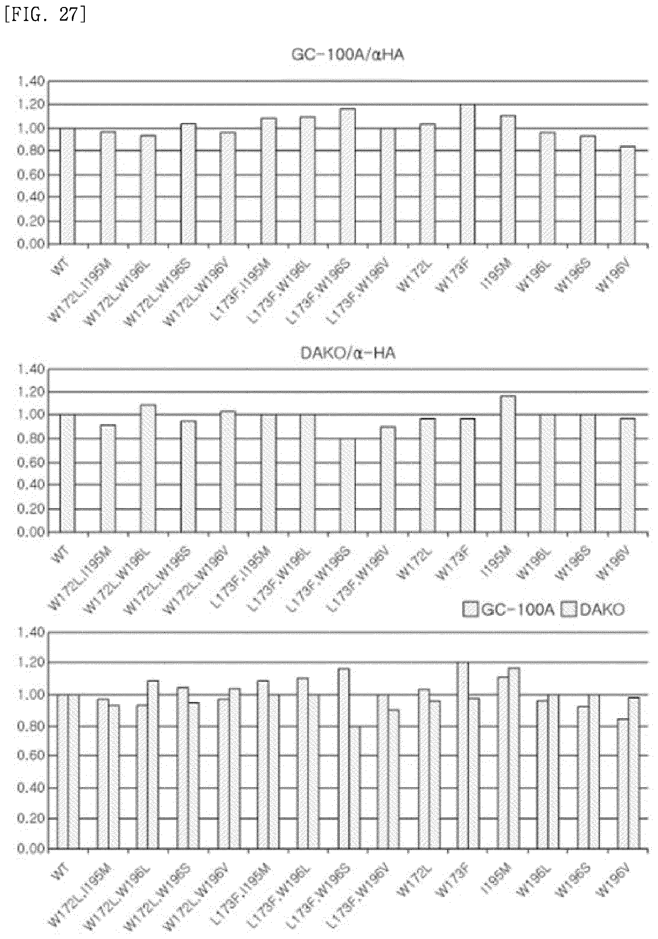

In addition, the antibody or the fragment thereof, according to the present invention, can bind to hepatitis B virus that is resistant to the therapeutic drugs for hepatitis B virus on the market or under development, such as telbivudine, tenofovir, lamivudine, adefovir, clevudine, or entecavir, and thus have neutralizing activity thereagainst (Experimental Example 4 and FIGS. 24 to 27).

In addition, the antibody or the fragment thereof, according to the present invention, can bind to HBsAg with a mutation at amino acid position 80, 101, 112, 126, 129, 133, 143, 172, 173, 175, 181, 184, 185, 195, 196, 204, or 236, and thus have neutralizing activity against mutated hepatitis B virus. In an embodiment of the present invention, the mutated HBsAg may be an antigen with a mutation at position 80, 171, 172, 173, 195, 196, 204, or 236 of HBsAg. However, the antigen is not limited thereto.

In still yet another aspect of the present invention, there is provided a pharmaceutical composition for treating HBV infection, comprising, as an active ingredient, the hepatitis B virus-neutralizing antibody or the fragment thereof.

Here, the pharmaceutical composition may further comprise a pharmaceutically acceptable carrier. For the pharmaceutically acceptable carrier, it is possible to use any carrier as long as it is a non-toxic material suitable for delivery to a patient. Examples of the carrier may include distilled water, alcohols, fats, waxes, and inert solids. A pharmaceutically acceptable adjuvant (buffer or dispersant) may also be included in the pharmaceutical composition.

Specifically, the pharmaceutical composition of the present application may comprise, in addition to the active ingredient, a pharmaceutically acceptable carrier, and thus be prepared into a parenteral formulation, depending on the administrate route, using any conventional method known in the art. Here, the term "pharmaceutically acceptable" refers to a substance that does not inhibit activity of the active ingredient and does not have toxicity beyond that to which a subject receiving the same can adapt.

In a case where the pharmaceutical composition of the present application is prepared into a parenteral formulation, it may be formulated in the form of an injection, a drug for transdermal delivery, a drug for nasal inhalation, or a suppository, together with a suitable carrier, according to any method known in the art. In a case where the pharmaceutical composition is formulated as an injection, the suitable carrier used may include sterile water, ethanol, polyol such as glycerol or propylene glycol, or any mixture thereof, and may preferably include Ringer's solution, sterile water for injection or phosphate buffered saline (PBS) containing triethanolamine, an isotonic solution such as 5% dextrose, or the like. Methods related to formulation of the pharmaceutical composition are known in the art.

A preferred dosage of the pharmaceutical composition of the present application may be in a range of 0.01 ug/kg to 10 g/kg per day, or a range of 0.01 mg/kg to 1 g/kg per day, depending on the patient's condition, body weight, sex, age, disease severity, and route of administration. Administration may be performed once a day or divided into several times. Such dosages should not be construed as limiting the scope of the present invention in any way.

The subject to which the composition of the present application can be applied includes mammals and humans, with humans being particularly preferred. In addition to the active ingredient, the pharmaceutical composition of the present application may further comprise any compound or natural extract, which has already been verified for safety and is known to have a therapeutic effect on infectious diseases, in order to increase or reinforce therapeutic activity thereof.

In still yet another aspect of the present invention, there is provided a use of the hepatitis B virus-neutralizing antibody or the fragment thereof, for manufacture of a pharmaceutical composition for treating HBV infection.

In still yet another aspect of the present invention, there is provided a method for treating HBV infection, comprising a step of administering, to an individual, an effective amount of the pharmaceutical composition.

In still yet another aspect of the present invention, there is provided a method for producing the hepatitis B virus-neutralizing antibody or the fragment thereof, which specifically binds to the above-described HBsAg conformational epitope, comprising steps of: 1) measuring binding capacity of antibodies specific for HBV to the above-described HBsAg conformational epitope; 2) measuring binding capacity of the antibodies specific for HBV to a modified version of the above-described HBsAg conformational epitope, which has been obtained by substituting any one amino acid residue selected from the group consisting of Thr, A₁, A₂, A₃, A₄, A₅, and A₆ of General Formula 1 with another residue in the above-described HBsAg conformational epitope; and 3) selecting an antibody whose binding capacity to the HBsAg conformational epitope measured in step 1) is strong as compared with its binding capacity to the modified version of the HBsAg conformational epitope measured in step 2).

Here, in the amino acid residue substitution mentioned in step 2), A₁ may be substituted with an amino acid other than Lys or Arg. In addition, A₂ may be substituted with an amino acid other than Thr, Ala, Ile, Asn, or Ser. In addition, A₃ may be substituted with an amino acid other than Ser or Leu. In addition, A₄ may be substituted with an amino acid other than Arg or His, and A₅ may be substituted with an amino acid other than Ser or Phe. In addition, A₆ may be substituted with an amino acid other than Ser or Asn.

### Mode for the Invention

Hereinafter, the present invention will be described in more detail by way of the following examples. However, the following examples are for illustrative purposes only, and the scope of the present invention is not limited thereto.

### Preparation Example 1. Preparation of antibody that specifically binds to HBsAg

In the present invention, for the antibody that specifically binds to HBsAg, a monoclonal antibody produced by the strain having accession number KCTC13760BP was used. In addition, the monoclonal antibody produced by the strain was designated, for convenience, as GC-100A.

### Experimental method 1. Native agarose gel electrophoresis (NAGE)

Huh-7 cells were transfected with a mock vector or a flag-small HBsAg-expressing plasmid using Lipofectamine 3000. After 2 days, the cells were lysed using RIPA buffer (Thermo Fisher, 89901). Centrifugation was performed at 4°C and 12,000 rpm for 15 minutes, and then the supernatant without pellet was transferred to a new Eppendorf tube. 6X agarose loading dye (50% glycerol, 0.1% BPB) was added thereto to the final 1X concentration, and mixed well. A 1.2% TBE agarose gel was prepared, and a sample mixed with the agarose loading dye was loaded thereon. Then, gel electrophoresis was performed at 50 V for 1 hour.

After the gel electrophoresis was completed, an upward capillary transfer was performed on a polyvinylidene difluoride membrane (PVDF membrane) using 20X SSC buffer (3 M NaCl, 0.3 M sodium citrate, pH 7.2). The membrane, on which the material transfer was completed, was blocked with 5% skim milk solution for 30 minutes, and washed twice using 1X TBST buffer (50 mM Tris, 150 mM NaCl, 0.1% Tween 20). Subsequently, GC-100A or an anti-Flag antibody (5 ug) was diluted in 10 ml of TBS blocking buffer (Thermo Fisher, 37571), and then incubated with the membrane for 2 hours on a shaker. After the incubation was completed, washing with 1X TBST buffer was performed for 5 minutes on a shaker. This process was repeated 4 times.

Anti-human antibodies or anti-mouse antibodies were diluted in 10 ml of TBS blocking buffer, and then incubated with the membrane for 1 hour on a shaker. Finally, as described above, washing with 1X TBST buffer was performed for 5 minutes on a shaker. This process was repeated 4 times. The membrane, on which reaction with primary antibodies and secondary antibodies was completed, was reacted with ECL (GE healthcare, RPN2235). Then, images were obtained using the ChemiDoc MP system (Bio-rad, 1708280).

### Experimental method 2. Denatured immunoprecipitation experiment

HEK293T cells were transfected with a mock vector or a flag-small HBsAg-expressing plasmid using Lipofectamine 3000. After 2 days, the cells were lysed using NP40 cell lysis buffer (150 mM NaCl, 50 mM Tris-HCl pH 7.5, 1% NP-40). Centrifugation was performed at 4°C and 12,000 rpm for 15 minutes, and then the supernatant without pellet was transferred to a new Eppendorf tube.

The cell lysate was divided into 4 samples; and then 2 of them were subjected to native IP and the other 2 were subjected to denaturation (heated at 100°C for 10 minutes, with final concentration being 1% SDS, 1% beta-mercaptoethanol). Each of the 4 samples was transferred to a 15 ml conical tube, and then diluted 20-fold using NP40 cell lysis buffer. To match the conditions under which antibody binding occurs in the native samples and the denatured samples, 0.05% SDS and 0.05% beta-mercaptoethanol at final concentrations were added to the 2 native samples. 5 ug of anti-Flag antibody was added to each one of the native and denatured samples, and 5 ug of GC-100A was added to each one of the remaining non-denatured and denatured samples. Then, incubation was performed overnight with shaking in a cold room at 4°C.

The next day, 100 ul each of protein A sepharose bead (GE healthcare, 17-0780-01) was added thereto, and incubation was performed again for 5 hours with shaking in a cold room. For each sample, after the reaction was completed, centrifugation was performed at 4°C and 2,000 rpm for 3 minutes. The supernatant was discarded, and the remaining beads were transferred to a new Eppendorf tube using NP40 cell lysis buffer. Centrifugation was performed again at 4°C and 4,000 rpm for 3 minutes. The supernatant was discarded, and washing was performed again using 500 ul of NP40 cell lysis buffer. This process was repeated 4 times. The supernatant was completely discarded. 50 ul of 1.5X sample buffer (obtained by adding 4% beta-mercaptoethanol at a final concentration to NP0007 (Thermo Fisher) and performing dilution at 1.5X) was added thereto, and boiling was performed at 100°C. Subsequently, centrifugation was performed at 4°C and 12,000 rpm for 3 minutes, and then the supernatant was transferred to a new Eppendorf tube.

Each of the above samples was loaded on a 12% Bis-Tris protein gel (Thermo Fisher, NP0342BOX), and gel electrophoresis was performed at 110 V for 2 hours (until the dye was electrophoresed to the end of the gel). After the gel electrophoresis was completed, the materials were transferred to a PVDF membrane. The membrane, on which the material transfer was completed, was blocked with 5% skim milk solution for 30 minutes, and washed twice using 1X TBST buffer (50 mM Tris, 150 mM NaCl, 0.1% Tween 20). Subsequently, GC-100A or an anti-Flag antibody (5 ug) was diluted in 10 ml of TBS blocking buffer (Thermo Fisher, 37571), and then incubated with the membrane for 2 hours on a shaker.

After the incubation was completed, washing with 1X TBST buffer was performed for 5 minutes on a shaker. This process was repeated 4 times. Anti-human antibodies or anti-mouse antibodies were diluted in 10 ml of TBS blocking buffer, and then incubated with the membrane for 1 hour on a shaker. Finally, as described above, washing with 1X TBST buffer was performed for 5 minutes on a shaker. This process was repeated 4 times. The membrane, on which reaction with primary antibodies and secondary antibodies was completed, was reacted with ECL (GE healthcare, RPN2235). Then, images were obtained using the ChemiDoc MP system (Bio-rad, 1708280).

### Experimental method 3. Epitope mapping between HBsAg and GC-100A

To form an immune complex, HBsAg VLP was mixed with GC-100A Fab as shown in Table 1.

**[Table 1]**

| | HBsAg VLP | | GC-100A Fab | | Mix HBsAg VLP/ GC-100A Fab | |
|---|---|---|---|---|---|---|
| mix | Volume | Cone. | Volume | Cone. | Volume | Cone. |
| VLP/Fab | 5 µl | 1mg/mL | 5 µl | 2 mg/mL | 10 µl | 500 µg/mL/1 mg/mL µM |

1 mg of d0 cross-linker was mixed with 1 mg of d12 cross-liker. Again, the mixture was mixed with 1 ml of DMF to obtain a 2 mg/ml solution of DSS d0/d12. 10 ul of the previously prepared antibody/antigen mixture was mixed with 1 ul of the solution of cross-linker d0/d12. For the crosslinking reaction, this solution was incubated for 180 minutes at room temperature.

### Experimental Example 1. Identification of HBsAg epitope of GC-100A antibody

### Experimental Example 1.1. Identification of possibility to detect HBsAg VLP using GC-100A

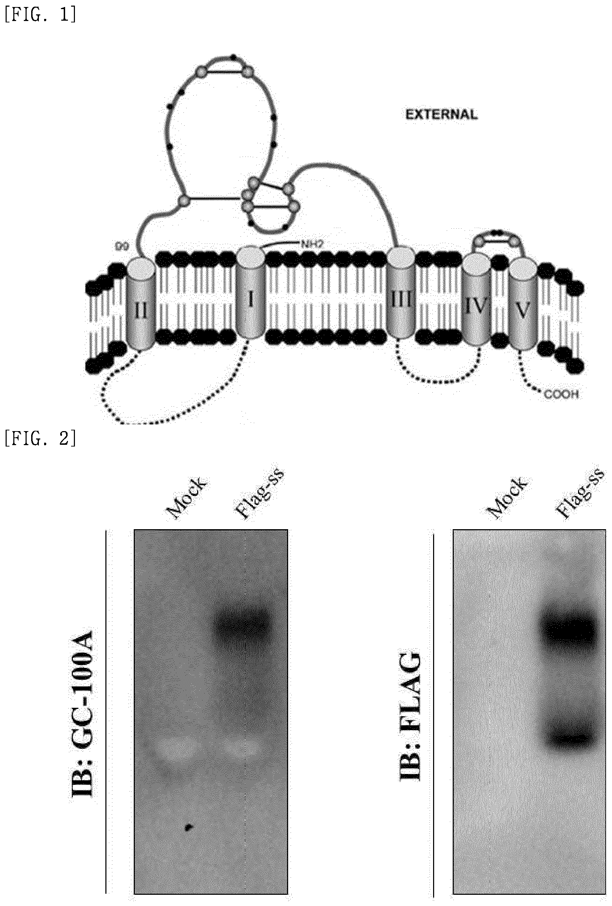

Until 2016, ELISA was the only test method that made it possible to detect HBsAg with GC-100A. In addition, in Western blot analysis using SDS-PAGE, it was not possible to detect HBsAg with GC-100A that is considered to have a conformational epitope. Accordingly, a technique of detecting HBV capsid in a native state was devised, and an attempt was made to detect HBsAg VLP (consisting of 100 single HBsAg molecules) using the NAGE disclosed in Experimental Method 1. Since the NAGE is a method of identifying large molecules without causing denaturation in proteins, this method made it possible to detect HBsAg VLP with an anti-Flag antibody having a linear epitope as well as GC-100A having a conformational epitope (FIG. 2).

There was a difference between the HBsAg VLP patterns detected by GC-100A and the anti-Flag antibody. In each upper band, it can be said that the VLP was in an intact state which is desired herein. On the other hand, in the lower band of the HBsAg VLP detected by the anti-Flag antibody, it was expected that the VLP would be in a denatured state. The reason for this was as follows. In the HBsAg VLP, lipid components account for about 30% or more; and thus it was expected that in a step of obtaining a cell lysate in the NAGE, the VLP lipid would be broken by Triton X-100, which is a detergent in RIPA buffer, to yield a complex.

Therefore, since GC-100A having a conformational epitope recognizes the VLP structure, it is possible to only detect the upper band where VLP is in an intact state. However, in a case of the anti-Flag antibody having a linear epitope, it is possible to detect VLP that is in a slightly broken state. In conclusion, in a case of using the NAGE without causing protein denaturation in HBsAg VLP, it was possible to detect the VLP with GC-100A (FIG. 2).

### Experimental Example 1.2. Identification on whether binding site of GC-100A is conformational epitope

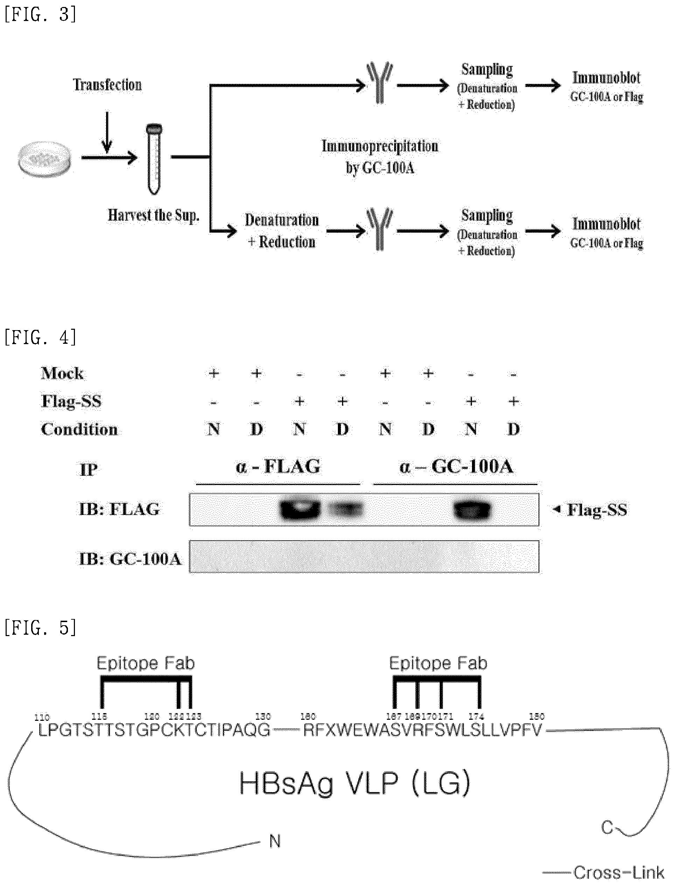

To identify whether the binding site of GC-100A has a conformational epitope, denatured IP was performed. According to the results of Experimental Example 1.1., it was possible to detect native HBsAg VLP with both GC-100A and the anti-Flag antibody. Therefore, GC-100A binds to native HBsAg VLP; however, in a case where the HBsAg VLP is denatured, GC-100A having a conformational epitope is not capable of binding thereto. In addition, in SDS-PAGE, GC-100A failed to detect HBsAg. Thus, the last detection was intended to be performed using the anti-Flag antibody. In other words, a method was selected in which binding (IP) with native or denatured HBsAg VLP is performed with GC-100A and detection (IB) of HBsAg VLP bound to GC-100A is performed with the anti-Flag antibody. An overview of the experiment is as illustrated in FIG. 3.

When immunoprecipitation (IP) was performed with GC-100A, GC-100A bound to the native HBsAg VLP. However, the denatured HBsAg VLP failed to bind to GC-100A (FIG. 4). The anti-Flag antibody having a linear epitope bound to the denatured HBsAg VLP as well as the native HBsAg VLP. The results obtained by performing denatured IP with the anti-Flag antibody indicate a control showing that the conditions were enough for the antibody to bind to the antigen. However, GC-100A failed to bind to the denatured HBsAg VLP, and this result supports that GC-100A has a conformational epitope.

In addition, unlike a case where immunoblotting (IB) was performed with the anti-Flag antibody, no band was observed in a case where IB was performed with GC-100A; and this becomes a separate supporting basis from the results as described above. Therefore, from the results obtained by conducting a denatured IP experiment, it was found that GC-100A bound to a conformational epitope.

### Experimental Example 2. Identification of epitope position of present antibody using protease

This experiment was conducted as follows. Two proteins were allowed to react naturally, and then cross-linkers were used to achieve cross-linking at sites close to each other. Then, protease was used to cut the proteins into peptide fragments so as to find the cross-linked sites.

Specifically, 1 mg of d0 cross-linker was mixed with 1 mg of d12 cross-liker, and then the mixture was mixed with 1 ml of DMF to obtain a 2 mg/ml solution of DSS d0/d12. 1 ul of the solution was mixed with 10 ul of GC-100A/HBsAg VLP complex, and incubated at room temperature for 180 minutes.

Then, the denatured, GC-100A/HBsAg VLP complex cross-linked with d0/d12 was digested with trypsin (Roche Diagnostic) that cleaves a lysine or arginine residue, and then 9 cross-linked peptides between Fab's, which are fragments of GC-100A, and HBsAg VLP were identified through nLC-orbitrap MS/MS analysis. These cross-linked peptide portions were detected by both Xquest and Stavrox software (Table 2).

**[Table 2]**

| **Sequence** | **Protein 1** | **Protein 2** | **Sequence Protein 1** | **Sequence Protein 2** | **XL Type** | **nAA1** | **nAA2** |
|---|---|---|---|---|---|---|---|
| GLEWVSSISSTSRDIDY ADSVK-FXWEWASVR -a9-b7 (SEQ ID NO: 4) | Antibody _HC | VLP | 44-65 | 161-169 | inter-protein xl | 52 | 167 |
| GLEWVSSISSTSR-LPV WPLLPGTSTTSTGPCK -a9-b12 (SEQ ID NO: 5) | Antibody _HC | VLP | 44-56 | 104-122 | inter-protein xl | 52 | 115 |
| LLLYSTSTLLSGVP SR-FXWEWASVR -a6-b7 (SEQ ID NO: 6) | Antibody _LC | VLP | 46-61 | 161-169 | inter-protein xl | 51 | 167 |
| LLLYSTSTLLSGVP SR-FXWEWASVR -a7-b7 (SEQ ID NO: 7) | Antibody _LC | VLP | 46-61 | 161-169 | inter-protein xl | 52 | 167 |
| LLLYSTSTLLSGVP SR-FXWEWASVR -a8-b7 (SEQ ID NO: 8) | Antibody _LC | VLP | 46-61 | 161-169 | inter-protein xl | 53 | 167 |
| LSCSASGFSLTKYK- LPV WPLLPGTSTTSTGPCK -a9-b12 (SEQ ID NO: 9) | Antibody _HC | VLP | 20-33 | 104-122 | inter-protein xl | 28 | 115 |
| LSCSASGFSLTKYKMT WVR-FXWEWASVR -a9-b7 (SEQ ID NO: 10) | Antibody _HC | VLP | 20-38 | 161-169 | inter-protein xl | 28 | 167 |
| LSCSASGFSLTKYKXT WVR-FXWEWASVR -a9-b7 (SEQ ID NO: 11) | Antibody _HC | VLP | 20-38 | 161-169 | inter-protein xl | 28 | 167 |
| QAPGKGLEWVSSISS TSR-FXWEWASVR -a12-b7 (SEQ ID NO: 12) | Antibody _HC | VLP | 39-56 | 161-169 | inter-protein xl | 50 | 167 |

The denatured, GC-100A/HBsAg VLP complex cross-linked with d0/d12, which was prepared by the same preparation method as described above, was digested with chymotrypsin (Roche Diagnostic) that cleaves tryptophan, tyrosine, phenylalanine, leucine, and methionine residues. Then, one cross-linked peptide between Fab's and HBsAg VLP was detected through nLC-orbitrap MS/MS analysis. These cross-linked peptide portions were detected by both Xquest and Stavrox software (Table 3).

**[Table 3]**

| **Sequence** | **Protein 1** | **Protein 2** | **Sequence Protein 1** | **Sequence Protein 2** | **XL Type** | **nAA1** | **nAA2** |
|---|---|---|---|---|---|---|---|
| FVTPETFGQGTKLEIKRT VAAPSVF-SLSSVPF -a11-b2 (SEQ ID NO: 13) | Antibody _LC | VLP | 92-116 | 173-179 | inter-protein xl | 102 | 174 |

The denatured, GC-100A/HBsAg VLP complex cross-linked with d0/d12, which was prepared by the same preparation method as described above, was digested with ASP-N enzyme (Roche Diagnostic) that cleaves aspartic acid and glutamic acid. Then, nLC-orbitrap MS/MS analysis was performed; however, no cross-linked peptide between Fab's and HBsAg VLP was detected. The GC-100A/HBsAg VLP complex was digested with elastase (Roche Diagnostic) that cleaves serine using the same method as described above, and then 5 cross-linked peptides between Fab's and HBsAg VLP were detected through nLC-orbitrap MS/MS analysis. These cross-linked peptide portions were detected by both Xquest and Stavrox software (Table 4).

**[Table 4]**

| **Sequence** | **Protein** 1 | **Protein 2** | **Sequence Protein 1** | **Sequence Protein 2** | **XL Type** | **nAA1** | **nAA2** |
|---|---|---|---|---|---|---|---|
| TKYKXTWV-T GPCKTCTIPA -a4-b6 (SEQ ID NO: 14) | Antibody _HC | VLP | 30-37 | 118-128 | inter-protein xl | 33 | 123 |
| VPSRFSG -RFSWL -a4-b3 (SEQ ID NO: 15) | Antibody _LC | VLP | 58-64 | 169-173 | inter-protein xl | 61 | 171 |
| KGRFTI-TTSTG PCKTCTIPAQG -a3-b8 (SEQ ID NO: 16) | Antibody _HC | VLP | 65-70 | 115-130 | inter-protein xl | 67 | 122 |
| FSLTKYKXTWV-TS TTSTGPCKTCTIPA -a6-b10 (SEQ ID NO: 17) | Antibody _HC | VLP | 27-37 | 113-128 | inter-protein xl | 32 | 122 |
| TKYKXTWVRQ | Antibody | VLP | 30-44 | 167-171 | inter-protein xl | 35 | 169 |
| APGKG-SVRFS -a6-b3 (SEQ ID NO: 18) | _HC | | | | | | |

The GC-100A/HBsAg VLP complex was digested with thermolysin (Roche Diagnostic) that cleaves a hydrophobic amino acid residue using the same method as described above, and then one cross-linked peptide between Fab's and HBsAg VLP was detected through nLC-orbitrap MS/MS analysis. These cross-linked peptide portions were detected by both Xquest and Stavrox software (Table 5).

**[Table 5]**

| **Sequence** | **Protein 1** | **Protein 2** | **Sequence Protein 1** | **Sequence Protein 2** | **XL Type** | **nAA1** | **nAA2** |
|---|---|---|---|---|---|---|---|
| LSCSASGFSLTKYKMT WVR-FXWEWASVR -a9-b7 (SEQ ID NO: 19) | Antibody _HC | VLP | 20-38 | 161-169 | inter-protein xl | 28 | 167 |

Specifically, protein 1 is a heavy chain and protein 2 is VLP; and sequence proteins 1 and 2 are peptide portions bound thereto. nAA1 and nAA2 at the end are portions connected by a cross-linker. The data obtained so far were used to infer an epitope of GC-100A. As a result, it was identified that the epitope included two parts, one being at amino acid residues 115, 122, and 123 of HBsAg and the other one being at amino acid residues 167, 169, 171, and 174 of HBsAg (FIG. 5).

### Experimental Example 3. Examination of binding capacity of GC-100A to HBsAg mutant

GC-100A binds to a conformational epitope of HBsAg, and thus may have different binding capacity to HBsAg depending on types of HBsAg and mutations therein. Therefore, several types of HBsAg mutants were constructed, and binding capacity of GC-100A thereto was examined.

### Experimental Example 3.1. Examination of binding capacity of GC-100A to clinical ('a' determinant) variants

Among the clinical variants reported to date (Alavian SM, J Clinical Virol. 57:201, 2013), in consideration of the membrane distribution structure of HBsAg (Rezaee R. et al., Hepat Mon. 16:e39097, 2016) with a focus being placed on the 'a' determinant region (amino acids 120 to 150), 30 point mutants for 16 major amino acid positions, such as G145R that is known as a vaccine escape mutant, were selected as primary analysis targets.

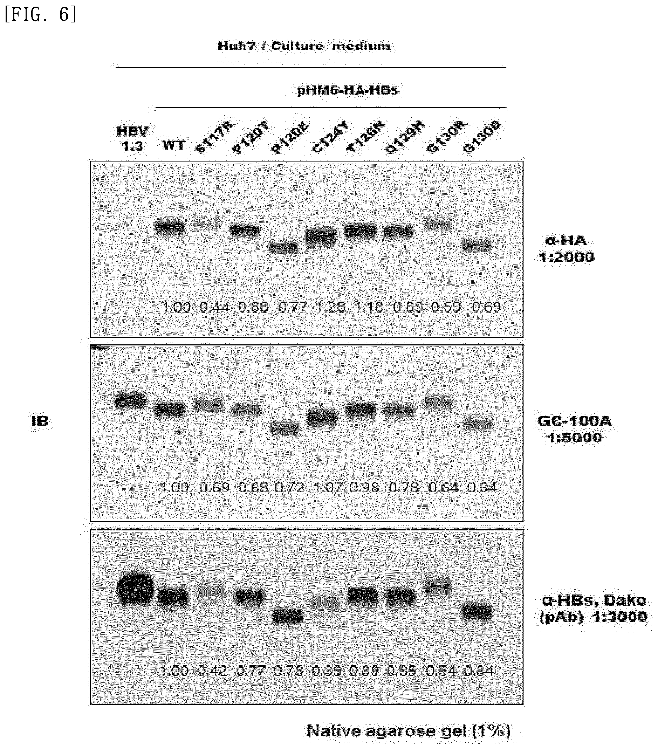

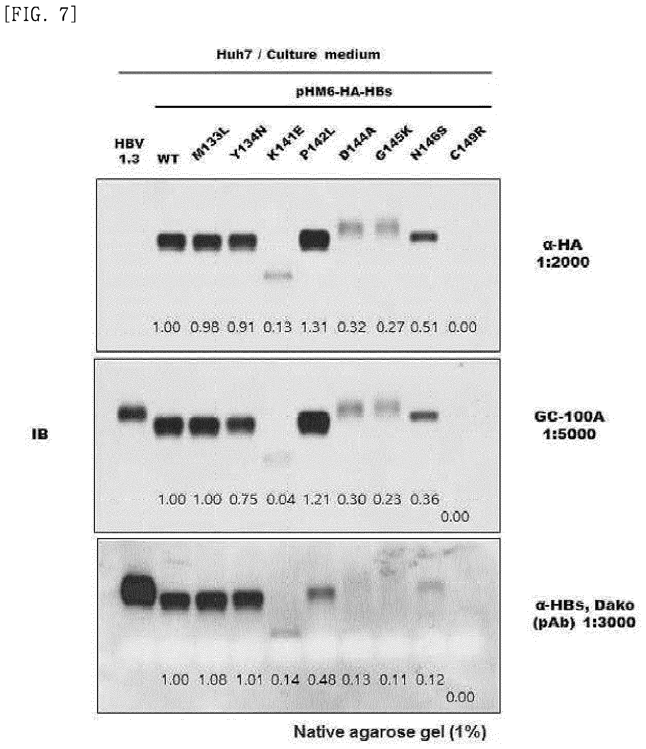

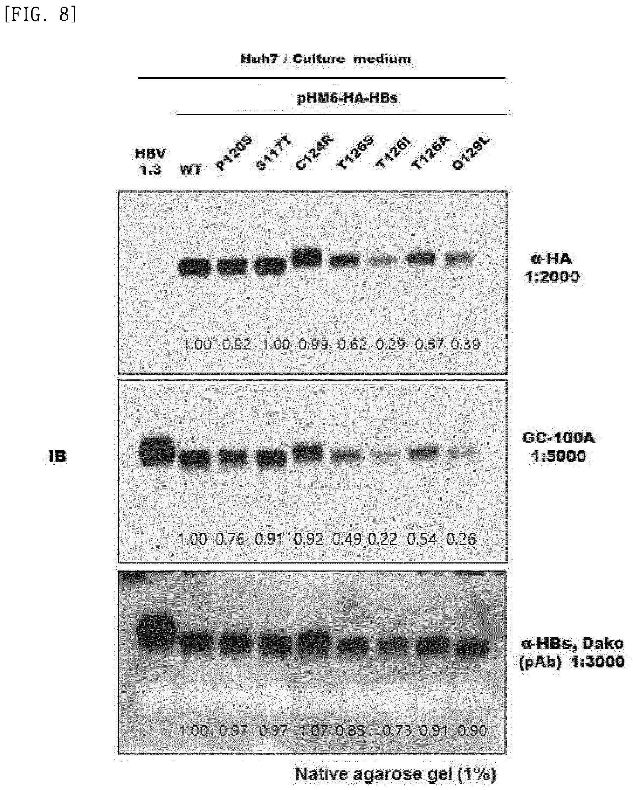

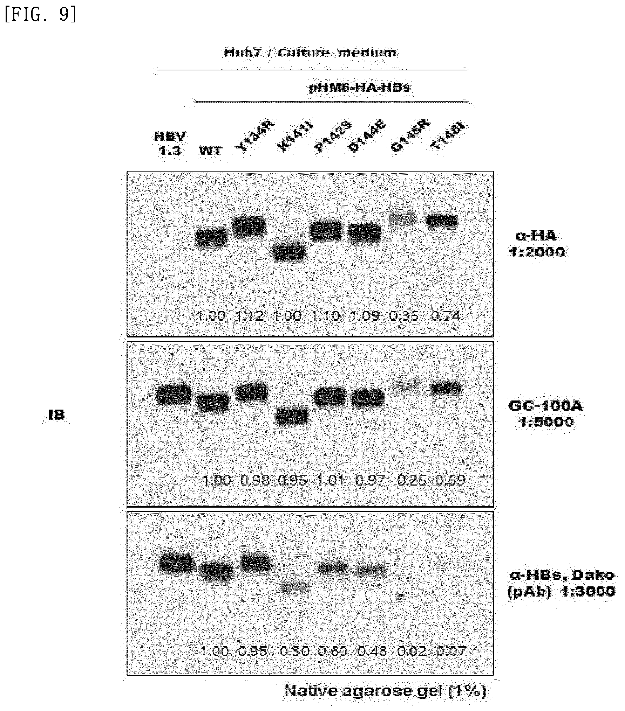

For the data obtained through NAGE in Experimental Method 1, the band intensity was measured with ImageJ software to determine the degree of binding of each anti-HBsAg antibody relative to the binding capacity of anti-HA. As a result, it was identified that there was a significant difference in terms of antigen-binding properties between the antibodies GC-100A and DAKO (anti-HBsAg polyclonal antibody) (FIGS. 6 to 9). Table 6 below lists the clinical mutants of HBsAg.

**[Table 6]**

| **Amino acid position** | **Wild-type (ayw)** | **Mutant** |
|---|---|---|
| 117 | S | R/T |
| 120 | P | E/S/T |
| 123 | T | N |
| 124 | C | R/Y |
| 126 | T | I/A/N/S |
| 129 | Q | H/L |
| 130 | G | D/R |
| 133 | M | L |
| 134 | Y | N/R |
| 141 | K | E/I |
| 142 | P | S/L |
| 144 | D | A/E |
| 145 | G | R/K |
| 146 | N | S |
| 148 | T | I |
| 149 | C | R |

As illustrated in FIGS. 6 to 9, the bands for HBsAg mutants have different positions in the NAGE. In this phenomenon, proteins having a substitution with a basic amino acid, such as S117R and G130R, migrated less. On the contrary, clones having a substitution with an acidic amino acid, such as P120E and G130D, migrated further. From these results, it was found that in the above NAGE, the charge of the protein was an important migration factor. Some clones, such as S117R and G130R, exhibited no or a significantly lower antigenic protein level as compared with WT. In view of the fact that in a case of being examined with the anti-HA antibody as described above, such clones exhibited an intracellular protein expression level which was not remarkably lower than the other clones, it is determined that these mutants have low efficiency in secreting proteins to the outside of the cell or the proteins secreted therefrom have low stability. The GC-100A showed approximately similar binding capacity to 30 clinical ('a' determinant) variants. On the other hand, the DAKO antibody, which was produced against the patient's blood-derived HBsAg antigen, showed greatly low binding capacity, in terms of antigen binding, to all 10 mutants ranging from K141E to T148I; and this result was remarkably distinguished from that for GC-100A.

The portion composed of about 10 amino acids is at a location corresponding to the second loop of the 'a' determinant, and seems to be very important for binding of the DAKO antibody. On the other hand, unlike most of the 'a' determinant-dependent anti-HBsAg antibodies, it is determined that GC-100A showed fairly consistent binding to various clinical variants including vaccine escape mutants such as G145K or G145R (FIG. 10). Specifically, it seems that binding of the DAKO antibody weakened at positions 141 to 149. This can represent vaccine escape mutants. On the contrary, GC-100A showed no difference in binding capacity at the same positions, from which it was predicted that GC-100A would be effective against the vaccine escape mutants. Here, the vaccine escape mutant means a case of causing HBV infection again in subjects who have received HBV vaccination and produced antibodies.

### Experimental Example 3.2. Identification of efficacy of GC-100A against vaccine escape mutant G145R

Elimination abilities were checked in mice against the vaccine escape mutant G145R. For a short-term expression mouse model of the wild-type HBV or the vaccine escape mutant G145R, in the group treated with hIgG, which is a negative control, it was identified that HBsAg of the wild-type virus or the vaccine-avoidant mutant (G145R) was maintained in the blood until about day 6 (FIGS. 11 and 13). On the contrary, in the group treated with GC-100A, it was identified that HBsAg of the wild-type virus or the vaccine-avoidant mutant (G145R) decreased sharply on day 1 after treatment (FIGS. 12 and 14).

qPCR was performed to quantitatively analyze infectious virus particles (virions) other than viral HBsAg. As a result, in the group treated with GC-100A, it was identified that DNA of the wild-type virus or the vaccine-avoidant mutant (G145R) decreased sharply in the blood on day 1 after treatment. However, in the group treated with hIgG, which is a negative control, it was identified that DNA of the wild-type virus or the vaccine-avoidant mutant (G145R) in the blood was maintained until days 7 to 10 (FIGS. 15 to 18). Taking the above results together, it was identified that GC-100A could eliminate HBsAg and infectious virus particles (virions) present in the mouse blood, and this effect was observed in the vaccine escape mutant (G145R) as well as the wild-type virus.

### Experimental Example 3.3. Examination of binding capacity of GC-100A for genotypes/serotypes

HBV consists of 10 genotypes (A, B, C, D, E, F, G, H, I, and J) and 4 serotypes (ayw, ayr, adw, and adr). The binding capacity of GC-100A to various genotypes and serotypes of HBV was checked.

For the amino acid sequences that are representative for respective genotypes, several sequences derived from patient samples were compared and consensus sequences were selected. The number of the selected consensus sequences for each type was as follows: 2 for type A; 2 for type B; 2 for type C; 2 for type D; 2 for type E; 3 for type F; 2 for type G; and 3 for type H. Since the types I and J were recently isolated genotypes, there was not much sequence information. Thus, the sequences possessed by the present inventors were selected as representative sequences. In addition, since the serotypes adw, adr, and ayw existed in the 21 representative sequences for respective genotypes, the sequence of the serotype ayr, which was possessed by the present inventors, was added. Therefore, a total of 22 sequences were used to examine the binding capacity of GC-100A.

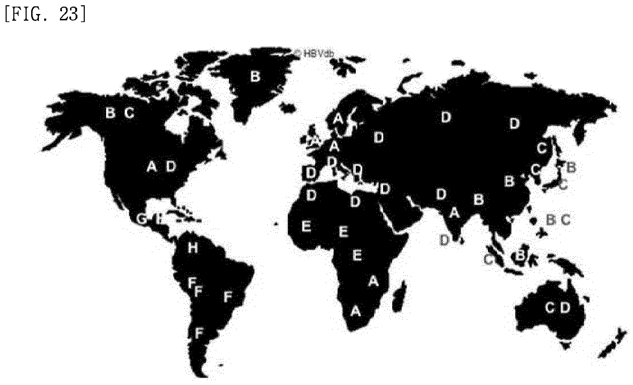

As a result of performing identification with the above method, it was shown that as compared with the DAKO antibody, GC-100A responded somewhat strongly to genotype C and somewhat weakly to genotypes F and H (FIGS. 19 to 22). These results show that GC-100A is capable of binding to various HBsAgs for all genotypes/serotypes. In addition, most of the patient's genotypes are A, B, C, and D; and the genotypes F and H are representative genotypes locally only in South America (FIG. 23).

### Experimental Example 4. Examination of binding capacity of GC-100A to drug-resistant variants

Currently, there are two types of FDA-approved antiviral drugs for chronic hepatitis B virus (CHB), that is, a nucloet(s)ide-based type and an interferon-a-related type. Among them, the nucleot(s)ide drug includes lamivudine, telbivudine, adefovir, tenofovir, and entecavir, and has a mechanism to act on HBV polymerase for prevention of viral replication. However, long-term administration of the nucleot(s)ide drug causes resistant mutants thereagainst, and thus the virus titer is restored again despite administration of the drug. Therefore, a combination treatment using the nucleot(s)ide drug has been conducted. However, this treatment becomes a big issue because such a treatment has caused multidrug resistance.

The relationship between the drugs acting on polymerase and GC-100A binding to HBsAg is as follows. HBV has a small genome of 3.2 kb, and thus polymerase ORF and HBsAg ORF overlap therein. Therefore, in a case where a mutation occurs in the polymerase, HBsAg also undergoes a mutation, which may affect binding of GC-100A. Therefore, the present inventors intended to examine binding capacity of GC-100A to these mutants.

An examination was performed on mutations in the polymerase caused by lamivudine, telbivudine, adefovir, tenofovir, entecavir, and multidrugs, and mutations in HBsAg resulting therefrom (Table 7).

**[Table 7]**

| **Drug** | **RT mutation** | **S mutation** |
|---|---|---|
| Lamivudine | A181T/V | W172L or L173F |
| | M204V/I/S | I195M or W196S/L/V |
| Telbivudine | A181T/V | W172L or L173F |
| | M204I | W196L/S |
| Adefovir/tenofovir | A181T/V | W172L or L173F |
| | N236T | No change |
| Entecavir | L180M | No change |
| | M204V/I | I195M or W196S/L |
| Multidrug | A181T/V | W172L or L173F |
| | M204V/I | I195M or W196S/L |
| | N236T | No change |

The mutation sites in the polymerase caused by the five RT inhibitors were 180L, 181A, 204M, and 236N in the polymerase, and the mutation in HBsAg resulting therefrom occurred at positions 172, 173, 195, or 196. The polymerase mutants become resistant in a case where a double mutation occurs; however, they become resistant even in a case where a single mutation occurs in one of the two sequences. Therefore, 14 combinations were created in consideration of combinations thereof. Table 8 below lists drug-resistant strains.

**[Table 8]**

| | Mutant constructs |
|---|---|
| 1 | W172L, I195M |
| 2 | W172L, W196L |
| 3 | W172L, W196S |
| 4 | W172L, W196V |
| 5 | L173F, I195M |
| 6 | L173F, W196L |
| 7 | L173F, W196S |
| 8 | L173F, W196V |
| 9 | W172L |
| 10 | L173F |
| 11 | I195M |
| 12 | W196L |
| 13 | W196S |
| 14 | W196V |

The binding capacity of GC-100A to the mutants was examined using NAGE. For all 14 constructs, GC-100A showed binding capacity thereto which is similar to WT (FIGS. 24 to 26). Therefore, GC-100A shows good binding irrespective of nucleot(s)ide resistant mutants, and thus can be applied to patients who are incurable due to generation of resistant mutants.

The antibody was deposited with the Korea Research Institute of Bioscience and Biotechnology under accession number KCTC13760BP on December 5, 2018.

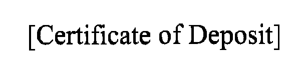

BUDAPEST TREATY ON THE INTERNATIONAL RECOGNITION OF THE
DEPOSIT
OF MICROORGANISMS FOR THE PURPOSES OF PATENT PROCEDURE

INTERNATIONAL FORM

RECEIPT IN THE CASE OF AN ORIGINAL DEPOSIT

issued pursuant to Rule 7.1 by the INTERNATIONAL DEPOSITARY AUTHORITY
identified at the bottom of this page
TO: Green Cross Corp.
107 Ihyeon-ro 30-beongil, Giheung-gu, Yongin-si, Gyeonggi-do 16924, Republic
of Korea

I. IDENTIFICATION OF THE MICROORGANISM
Identification reference given by the
DEPOSITOR:
CHO cell – GC100A | Accession number given by the
INTERNATIONAL DEPOSITARY
AUTHORITY:
KCTC13760BP
II. SCIENTIFIC DESCRIPTION AND/OR PROPOSED TAXONOMIC
DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under
I above, which was received by it
on December 5, 2018.
IV. RECEIPT OF REQUEST FOR CONVERSION
The microorganism identified under I above was received by this International
Depositary Authority on
and a request to convert the original deposit to a deposit under the
Budapest Treaty was received by it on.
V. INTERNATIONAL DEPOSITARY AUTHORITY
 |

Name: Korean Collection for Type Cultures (KCTC) | Signature(s) of person(s) having the power to represent the International Depositary Authority or of authorized official(s):
Address: Korea Research Institute of Bioscience and Biotechnology (KRIBB) 181, Ipsin-gil, Jeongeup-si, Jellabuk-do 56212 Republic of Korea | Sang-Gun Kim
Date: December 5, 2018

Form BP/4 (KCTC Form 17)

PCB912099GCC.txt
<110>    GREEN CROSS CORPORATION

<120>    CONFORMATIONAL EPITOPE OF HEPATITIS B VIRUS SURFACE ANTIGEN AND
         ANTIBODY SPECIFICALLY BINDING TO THE SAME

<130>    PCB912099GCC

<150>    KR 10-2019-0006092
<151>    2019-01-17

<160>    19

<170>    KoPatentIn 3.0

<210>    1
<211>    226
<212>    PRT
<213>    Artificial Sequence

<220>
<223>    HEPATITIS B VIRUS SURFACE ANTIGEN


<400>    1
Met Glu Ser Thr Thr Ser Gly Phe Leu Gly Pro Leu Leu Val Leu Gln
  1               5                  10                  15

Ala Gly Phe Phe Leu Leu Thr Arg Ile Leu Thr Ile Pro Gln Ser Leu
             20                  25                  30

Asp Ser Trp Trp Thr Ser Leu Asn Phe Leu Gly Gly Ala Pro Thr Cys
         35                  40                  45

Pro Gly Gln Asn Ser Gln Ser Pro Thr Ser Asn His Ser Pro Thr Ser
     50                  55                  60

Cys Pro Pro Ile Cys Pro Gly Tyr Arg Trp Met Cys Leu Arg Arg Phe
 65                  70                  75                  80

Ile Ile Phe Leu Phe Ile Leu Leu Leu Cys Leu Ile Phe Leu Leu Val
                 85                  90                  95

Leu Leu Asp Tyr Gln Gly Met Leu Pro Val Cys Pro Leu Leu Pro Gly
            100                 105                 110

Thr Ser Thr Thr Ser Thr Gly Pro Cys Lys Thr Cys Thr Thr Pro Ala
        115                 120                 125

Gln Gly Thr Ser Met Phe Pro Ser Cys Cys Cys Thr Lys Pro Ser Asp
    130                 135                 140

Gly Asn Cys Thr Cys Ile Pro Ile Pro Ser Ser Trp Ala Phe Ala Arg
145                 150                 155                 160

Phe Leu Trp Glu Trp Ala Ser Val Arg Phe Ser Trp Leu Ser Leu Leu
                165                 170                 175

Val Pro Phe Val Gln Trp Phe Ala Gly Leu Ser Pro Thr Val Trp Leu
            180                 185                 190

Ser Val Ile Trp Met Met Trp Tyr Trp Gly Pro Ser Leu Tyr Asn Ile
        195                 200                 205

Leu Ser Pro Phe Leu Pro Leu Leu Pro Ile Phe Phe Cys Leu Trp Val
    210                 215                 220

Tyr Ile
225

<210>    2
<211>    6
<212>    PRT
<213>    Artificial Sequence

<220>
<223>    (X1)6

<400>    2
Thr Ser Thr Gly Pro Cys
1               5

<210>    3
<211>    43
<212>    PRT
<213>    Artificial Sequence

<220>
<223>    (X2)43

<400>    3
Cys Thr Thr Pro Ala Gln Gly Thr Ser Met Phe Pro Ser Cys Cys Cys
1               5                   10                  15

Thr Lys Pro Ser Asp Gly Asn Cys Thr Cys Ile Pro Ile Pro Ser Ser
            20                  25                  30

Trp Ala Phe Ala Arg Phe Leu Trp Glu Trp Ala
        35                  40

<210>    4
<211>    31
<212>    PRT
<213>    Artificial Sequence

<220>
<223>    peptide sequence for analysis

<400>    4
Gly Leu Glu Trp Val Ser Ser Ile Ser Ser Thr Ser Arg Asp Ile Asp
1               5                   10                  15

Tyr Ala Asp Ser Val Lys Phe Xaa Trp Glu Trp Ala Ser Val Arg
            20                  25                  30

<210>    5
<211>    32
<212>    PRT
<213>    Artificial Sequence

<220>
<223>    peptide sequence for analysis

<400>    5
Gly Leu Glu Trp Val Ser Ser Ile Ser Ser Thr Ser Arg Leu Pro Val
1               5                   10                  15

Trp Pro Leu Leu Pro Gly Thr Ser Thr Thr Ser Thr Gly Pro Cys Lys
            20                  25                  30

<210> 6
<211> 25
<212> PRT
<213> Artificial Sequence

<220>
<223> peptide sequence for analysis

<400> 6
Leu Leu Leu Tyr Ser Thr Ser Thr Leu Leu Ser Gly Val Pro Ser Arg
1               5                   10                  15

Phe Xaa Trp Glu Trp Ala Ser Val Arg
            20                  25

<210> 7
<211> 25
<212> PRT
<213> Artificial Sequence

<220>
<223> peptide sequence for analysis

<400> 7
Leu Leu Leu Tyr Ser Thr Ser Thr Leu Leu Ser Gly Val Pro Ser Arg
1               5                   10                  15

Phe Xaa Trp Glu Trp Ala Ser Val Arg
            20                  25

<210> 8
<211> 25
<212> PRT
<213> Artificial Sequence

<220>
<223> peptide sequence for analysis

<400> 8
Leu Leu Leu Tyr Ser Thr Ser Thr Leu Leu Ser Gly Val Pro Ser Arg
1               5                   10                  15

Phe Xaa Trp Glu Trp Ala Ser Val Arg
            20                  25

<210> 9
<211> 33
<212> PRT
<213> Artificial Sequence

<220>
<223> peptide sequence for analysis

<400> 9
Leu Ser Cys Ser Ala Ser Gly Phe Ser Leu Thr Lys Tyr Lys Leu Pro

1               5                   10                  15

Val Trp Pro Leu Leu Pro Gly Thr Ser Thr Thr Ser Thr Gly Pro Cys
            20                  25                  30

Lys



<210>   10
<211>   28
<212>   PRT
<213>   Artificial Sequence

<220>
<223>   peptide sequence for analysis


<400>   10
Leu Ser Cys Ser Ala Ser Gly Phe Ser Leu Thr Lys Tyr Lys Met Thr
1               5                   10                  15

Trp Val Arg Phe Xaa Trp Glu Trp Ala Ser Val Arg
            20                  25


<210>   11
<211>   28
<212>   PRT
<213>   Artificial Sequence

<220>
<223>   peptide sequence for analysis


<400>   11
Leu Ser Cys Ser Ala Ser Gly Phe Ser Leu Thr Lys Tyr Lys Xaa Thr
1               5                   10                  15

Trp Val Arg Phe Xaa Trp Glu Trp Ala Ser Val Arg
            20                  25


<210>   12
<211>   27
<212>   PRT
<213>   Artificial Sequence

<220>
<223>   peptide sequence for analysis


<400>   12
Gln Ala Pro Gly Lys Gly Leu Glu Trp Val Ser Ser Ile Ser Ser Thr
1               5                   10                  15

Ser Arg Phe Xaa Trp Glu Trp Ala Ser Val Arg
            20                  25


<210>   13
<211>   32
<212>   PRT
<213>   Artificial Sequence

<220>
<223>   peptide sequence for analysis

<400>    13
Phe Val Thr Pro Glu Thr Phe Gly Gln Gly Thr Lys Leu Glu Ile Lys
1               5                   10                  15

Arg Thr Val Ala Ala Pro Ser Val Phe Ser Leu Ser Ser Val Pro Phe
            20                  25                  30

<210>    14
<211>    19
<212>    PRT
<213>    Artificial Sequence

<220>
<223>    peptide sequence for analysis

<400>    14
Thr Lys Tyr Lys Xaa Thr Trp Val Thr Gly Pro Cys Lys Thr Cys Thr
1               5                   10                  15

Ile Pro Ala

<210>    15
<211>    12
<212>    PRT
<213>    Artificial Sequence

<220>
<223>    peptide sequence for analysis

<400>    15
Val Pro Ser Arg Phe Ser Gly Arg Phe Ser Trp Leu
1               5                   10

<210>    16
<211>    22
<212>    PRT
<213>    Artificial Sequence

<220>
<223>    peptide sequence for analysis

<400>    16
Lys Gly Arg Phe Thr Ile Thr Thr Ser Thr Gly Pro Cys Lys Thr Cys
1               5                   10                  15

Thr Ile Pro Ala Gln Gly
            20

<210>    17
<211>    27
<212>    PRT
<213>    Artificial Sequence

<220>
<223>    peptide sequence for analysis

<400>    17
Phe Ser Leu Thr Lys Tyr Lys Xaa Thr Trp Val Thr Ser Thr Thr Ser
1               5                   10                  15

Thr Gly Pro Cys Lys Thr Cys Thr Ile Pro Ala
            20                  25


<210>    18
<211>    20
<212>    PRT
<213>    Artificial Sequence

<220>
<223>    peptide sequence for analysis


<400>    18
Thr Lys Tyr Lys Xaa Thr Trp Val Arg Gln Ala Pro Gly Lys Gly Ser
1               5                   10                  15

Val Arg Phe Ser
            20


<210>    19
<211>    28
<212>    PRT
<213>    Artificial Sequence

<220>
<223>    peptide sequence for analysis


<400>    19
Leu Ser Cys Ser Ala Ser Gly Phe Ser Leu Thr Lys Tyr Lys Met Thr
1               5                   10                  15

Trp Val Arg Phe Xaa Trp Glu Trp Ala Ser Val Arg
            20                  25

## Claims

1. A conformational epitope of hepatitis B surface antigen (HBsAg), comprising: a peptide represented by General Formula 1,
**General Formula 1** Thr-(X₁)ₙ₁-A₁-A₂-(X₂)ₙ₂-A₃-(X₃)ₙ₃-A₄-(X₄)ₙ₄-A₅-(X₅)ₙ₅-A₆
in the general formula, A₁ is Lys or Arg; A₂ is Thr, Ala, Ile, Asn, or Ser; A₃ is Ser or Leu; A₄ is Arg or His; A₅ is Ser or Phe; and A₆ is Ser or Asn; and
X₁ to X₅ are each independently a peptide molecule formed by bonding of n1 to n5 identical or different amino acids to each other, in which n1 is an integer from 4 to 8; n2 is an integer from 41 to 45; n3 is an integer from 0 to 2; n4 is an integer from 0 to 2; and n5 is an integer from 0 to 4.

2. The HBsAg conformational epitope of claim 1, wherein A₁ is Lys or Arg; A₂ is Thr; A₃ is Ser; A₄ is Arg; A₅ is Ser; and A₆ is Ser or Asn.

3. The HBsAg conformational epitope of claim 2, wherein A₁ is Lys, and A₆ is Ser.

4. The HBsAg conformational epitope of claim 1, wherein n1 is 6; n2 is 43; n3 is 1; n4 is 1; and n5 is 2.

5. An HBV vaccine, comprising as an active ingredient:
the HBsAg conformational epitope of any one of claims 1 to 4.

6. The HBV vaccine of claim 5, wherein the HBsAg conformational epitope is native.

7. A hepatitis B virus-neutralizing antibody or a fragment thereof, which specifically binds to the HBsAg conformational epitope of any one of claims 1 to 4.

8. The hepatitis B virus-neutralizing antibody or the fragment thereof of claim 7, wherein the neutralizing antibody or the fragment thereof has a therapeutic effect on infection with a vaccine escape mutant.

9. The hepatitis B virus-neutralizing antibody or the fragment thereof of claim 7, wherein the neutralizing antibody or the fragment thereof has a therapeutic effect on hepatitis B virus that is resistant to drugs such as telbivudine, tenofovir, lamivudine, adefovir, clevudine, or entecavir.

10. The hepatitis B virus-neutralizing antibody or the fragment thereof of claim 7, wherein the hepatitis B surface antigen (HBsAg) has the amino acid sequence of SEQ ID NO: 1.

11. The hepatitis B virus-neutralizing antibody or the fragment thereof of claim 7, wherein the antibody is produced in a cell line having accession number KCTC13760BP.

12. A pharmaceutical composition for treating HBV infection, comprising as an active ingredient:
the hepatitis B virus-neutralizing antibody or the fragment thereof of any one of claims 7 to 11.

13. A use of the hepatitis B virus-neutralizing antibody or the fragment thereof of any one of claims 7 to 11, for manufacture of a pharmaceutical composition for treating HBV infection.

14. A method for treating HBV infection, comprising:
a step of administering, to an individual, an effective amount of the pharmaceutical composition of claim 12.

15. A method for producing the hepatitis B virus-neutralizing antibody or the fragment thereof of claim 7, comprising steps of:
1) measuring binding capacity of antibodies specific for HBV to the HBsAg conformational epitope of any one of claims 1 to 4;
2) measuring binding capacity of the antibodies specific for HBV to a modified version of the HBsAg conformational epitope, which has been obtained by substituting any one amino acid residue selected from the group consisting of Thr, A₁, A₂, A₃, A₄, A₅, and A₆ of General Formula 1 with another residue in the HBsAg conformational epitope; and
3) selecting an antibody whose binding capacity to the HBsAg conformational epitope measured in step 1) is strong as compared with its binding capacity to the modified version of the HBsAg conformational epitope measured in step 2).

16. The method of claim 15, wherein in the amino acid residue substitution made in step 2),
A₁ may be substituted with an amino acid other than Lys or Arg;
A₂ may be substituted with an amino acid other than Thr, Ala, Ile, Asn, or Ser;
A₃ may be substituted with an amino acid other than Ser or Leu;
A₄ may be substituted with an amino acid other than Arg or His;
A₅ may be substituted with an amino acid other than Ser or Phe; and
A₆ may be substituted with an amino acid other than Ser or Asn.
